(19) Europäisches Patentamt
European Patent Office
Office européen des brevets

(11) **EP 0 686 196 B1**

(12) # FASCICULE DE BREVET EUROPEEN

(45) Date de publication et mention de la délivrance du brevet:
**23.08.2006 Bulletin 2006/34**

(21) Numéro de dépôt: **94908380.2**

(22) Date de dépôt: **25.02.1994**

(51) Int Cl.:
*C12N 15/82* (2006.01) *C12N 15/11* (2006.01)
*C12N 5/10* (2006.01) *A01H 5/00* (2006.01)
*A01N 63/00* (2006.01)

(86) Numéro de dépôt international:
**PCT/FR1994/000216**

(87) Numéro de publication internationale:
**WO 1994/019476 (01.09.1994 Gazette 1994/20)**

(54) **POLYRIBOZYME APTE A CONFERER, AUX PLANTES, UNE RESISTANCE AUX VIRUS ET PLANTES RESISTANTES PRODUISANT CE POLYRIBOZYME**

ZUR VERLEIHUNG VIRALER RESISTENZ AN PFLANZEN GEEIGNETES POLYRIBOZYM UND DIESES POLYRIBOZYM PRODUZIERENDE RESISTENTE PFLANZEN

PLANT VIRUS RESISTANCE CONFERRING POLYRIBOZYME AND RESISTANT PLANTS PRODUCING SAME

(84) Etats contractants désignés:
**AT BE CH DE DK ES FR GB GR IE IT LI LU MC NL PT SE**

(30) Priorité: **26.02.1993 FR 9302269**

(43) Date de publication de la demande:
**13.12.1995 Bulletin 1995/50**

(73) Titulaire: **Gene Shears Pty Limited**
**Canberra, ACT 2601 (AU)**

(72) Inventeurs:
- **LENEE, Philippe**
  **F-63110 Beaumont (FR)**
- **PEREZ, Pascual**
  **F-63110 Beaumont (FR)**
- **GRUBER, Véronique**
  **F-63400 Chamalières (FR)**
- **BAUDOT, Gaelle**
  **F-63000 Clermont-Ferrand (FR)**
- **OLLIVO, Catherine**
  **F-63200 Riom (FR)**

(74) Mandataire: **Almond-Martin, Carol et al**
**Ernest Gutmann - Yves Plasseraud S.A.S.**
**3, rue Auber**
**75009 Paris (FR)**

(56) Documents cités:
**EP-A- 0 321 201** **EP-A- 0 412 912**
**EP-A- 0 421 376** **WO-A-92/01786**
**WO-A-92/13089** **WO-A-93/02197**

- **NUCLEIC ACIDS RESEARCH vol. 20, no. 17 , 11 Septembre 1992 pages 4581 - 4589 CHEN, C-J, ET AL. 'Multitarget-ribozyme directed to cleave at up to nine highly conserved HIV-1 env RNA regions inhibits HIV-1 replication----potential effectivness against most presently sequenced HIV-1 isolates'**
- **RAVEN PRESS SERIES ON MOLECULAR AND CELLULAR BIOLOGY,VOL. 1. GENE REGULATION : BIOLOGY OF ANTISENSE RNA AND DNA 1992 pages 209 - 221 EDINGTON, B.V., ET AL. 'Utilization of ribozymes in plants. Plant viral resistance' cité dans la demande**
- **J. GEN. VIROL. vol. 71 , 1990 pages 2257 - 2264 LAMB, J.W., ET AL. 'Ribozymes that cleave potato leafroll virus RNA within the coat protein and polymerase genes' cité dans la demande**
- **Biological abstracts/rrm Document no. BR46: 67459 & J. CELL. BIOCHEM. SUPPL. vol. 18A , 1994 page 110 GRUBER, V., ET AL. 'Ribozymes and coat protein mediated CMV resistance in melon'**
- **ANTISENSE NUCLEIC ACIDS AND PROTEINS: FUNDAMENTALS AND APPLICATIONS. 1991 pages 157 - 168 GERLACH, W., ET AL. 'Ribozymes for the control of gene activity in vivo'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

(Cont. page suivante)

- STADLER GENETICS SYMPOSIUM SERIES: GENE MANIPULATION IN PLANT IMPROVEMENT, II. 19TH STADLER GENETICS SYMPOSIUM, MARCH 13-15, 1989. 1990 pages 313 - 330 YOUNG, M., ET AL. 'Using plant virus and related RNA sequences to control gene expression'
- BIOCHEMICAL AND BIOPHYSICAL RESEARCH COMMUNICATIONS vol. 189, no. 2 , 15 Décembre 1992 , DULUTH, MINNESOTA US pages 743 - 748 VON WEIZSÄCKER, F., ET AL. 'Cleavage of hepatitus B virus RNA by three ribozymes transcribed from a single DNA template'
- J. CELL. BIOCHEM. SUPPL. vol. 15A , 1991 page 59 NELSON, R.S., ET AL. 'Protection against tobacco mosaic virus challenge through expression of a ribozyme'
- CUOZZO BIO TECHNOLOGY vol. 6, 1988, pages 549 - 557
- PLAGES C.R. ACAD. AGRIC.FR. vol. 83, no. 1, 1997, pages 161 - 164
- HUTTNER CURR. ISSUES MOL BIOL vol. 3, no. 2, 2001, pages 27 - 34
- REZAIAN PLANT MOL BIOL vol. 11, 1988, pages 463 - 471

## Description

La présente invention concerne une séquence nucléotidique dite "polyribozyme" apte à conférer, aux plantes, une résistance aux virus, ainsi qu'un procédé pour rendre les plantes résistantes. L'invention vise également les plantes exprimant le polyribozyme.

Plusieurs approches ont été développées pour conférer, aux plantes cultivées, une résistance aux virus, en intégrant dans le génome des plantes des séquences d'acides nucléiques virales : le gène de la protéine de capside, les gènes de protéines non structurales, des séquences d'ARN viral en antisens, et des ARN de virus satellites (voir par exemple, Cuozzo et al, 1988, Bio/Technology 6, 549-557 ; Rezaian et al, 1988, Plant. Mol. Biol., 11, 463-471 ; Harrison et al, 1987, Nature 328, 797-802).

Ces travaux rapportent l'obtention de résistances partielles ou de tolérances. Néanmoins, il s'agit dans la plupart des cas de retard de symptômes, de symptômes atténués mais pas de résistance totale.

En outre, certaines de ces techniques, par exemple les ARN de virus satellites, peuvent donner lieu à de nouveaux problèmes. Par exemple, un ARN satellite réduisant les symptômes sur une espèce peut devenir léthal sur une autre espèce. De plus, des mutations sur les séquences nucléotidiques du virus satellite introduites dans les plantes peuvent augmenter la sévérité de l'infection au lieu de la diminuer.

De même, l'utilisation de la protéine de capside pour conférer une résistance présente des inconvénients. Par exemple, la protéine de capside d'une souche particulière du virus ne protège pas nécessairement la plante contre une infection par une autre souche de virus. Il est difficile d'utiliser le degré d'homologie de la séquence en acides aminés de la protéine de capside entre différents virus ou entre différentes souches pour prédire le niveau de tolérance permis par l'expression de la protéine. De plus, l'expression de protéines de capside pour protéger l'infection virale présente le risque d'induire des hétéroencapsidations entre la protéine de capside exprimée dans la plante et d'autres virus infectant la plante transgénique. Bien qu'elle n'ait jamais été démontrée pour des plantes transgéniques, cette hétéroencapsidation a déjà été observée entre deux souches de BYDV et entre le ZYMV et le PRSV.

L'utilisation de ribozymes a également été envisagée pour conférer, aux plantes, une résistance aux virus. Les ribozymes sont des molécules d'ARN qui agissent comme des enzymes en catalysant spécifiquement le clivage de l'ARN cible. Les premières expériences avec des ribozymes dans des cellules végétales ont été décrites dans la demande de brevet EP-A-321021. Depuis, plusieurs auteurs ont tenté d'optimiser la structure du ribozyme et les conditions opératoires, pour obtenir un clivage efficace de l'ARN viral.

Par exemple, Lamb et Hay (J. Gen. Virol., 1990, 71 : 2257-2264) ont démontré le clivage, in vitro, par des mono-ribozymes, de l'ARN du Virus de l'Enroulement de la Pomme de Terre (PLRV) dans des régions codant pour la RNA-polymérase et la protéine de capside. Ceci étant, la réaction de clivage in vitro n'a fonctionné qu'à 40°C ; aucune réaction n'a pu être observée à 0°C. Or les plantes sont généralement cultivées entre 10 et 30°C, selon l'espèce. Lamb et Hay suggèrent donc, pour une utilisation in vivo, d'augmenter la longueur des bras complémentaires. Ceci étant, une taille de bras trop importante peut conduire à la formation de duplex stable entre l'ARN cible et le ribozyme, empêchant la dissociation du ribozyme et le rendant incapable de catalyser une autre réaction de clivage. En outre, le ribozyme lui-même, en fonction de la longueur et de la séquence des bras complémentaires peut former des structures secondaires qui diminuent son activité de clivage.

Edington et Nelson ("Gene Regulation : biology of Antisense RNA and DNA : Ed. ERICKSON ET IZANT, Raven Press Ltd, New-York, 1992) ont décrit l'utilisation in vitro et in vivo de mono-ribozymes pour inactiver le gène de la polymérase du Virus de la Mosaïque du Tabac (TMV). Ils ont constaté que les ribozymes avaient un comportement très différent selon qu'ils étaient in vitro ou in vivo. L'activité d'un ribozyme in vitro ne peut donc pas être utilisée pour prévoir l'activité du même ribozyme in vivo. Par exemple, le clivage in vitro apparaît peu efficace et nécessite une concentration en ribozyme 20 fois supérieure à la concentration d'ARN génomique du TMV. Par contre, dans un expérience in vivo utilisant des protoplastes de tabac infectés par du TMV le ribozyme supprime 90 % de la multiplication de l'ARN viral. Il est intéressant de noter que l'ARN antisens utilisé comme témoin n'inhibe que de 20 % la multiplication virale. Ces auteurs font également référence à des travaux de Gerlach et al qui mettent en oeuvre un polyribozyme ciblé contre le gène de la polymérase du TMV. Ce polyribozyme n'a pas fonctionné in vitro en raison de la longueur du duplex formé entre le ribozyme et l'ARN cible. En revanche, in vivo, ce polyribozyme a clivé le substrat. Des plantes transgéniques de tabac exprimant, soit le monoribozyme, soit le polyribozyme ont montré, après infection par le TMV, un retard de symptômes. Une résistance totale, c'est-à-dire l'absence définitive de symptômes, n'est pas décrite. Les auteurs concluent que les paramètres comme la longueur optimale des bras complémentaires, le choix de la séquence cible et le choix du promoteur permettant de surmonter d'éventuels problèmes de "compartimentalisation" des ribozymes doivent être déterminés par l'expérience.

EP-A-0421376 décrit des ribozymes dirigés contre une séquence d'ARN non codante du CMV. WO-A-9213090 décrit l'inactivation de l'ARN de la protéine de capside du CMV par l'introduction, au sein de la séquence, d'une séquence hétérologue, en utilisant un monoribozyme du type "Groupe I intron". Aucun de ces documents ne décrit l'obtention d'une résistance totale contre le CMV.

Le problème technique que se propose de résoudre la présente invention est de fournir un moyen fiable et dépourvu d'inconvénients, de conférer aux plantes, une résistance aux virus.

Les présents inventeurs ont résolu ce problème par la conception et la mise en oeuvre d'un polyribozyme dirigé contre la protéine de capside d'un virus, ce polyribozyme est capable d'inactiver la gène codant pour cette protéine, et de conférer ainsi une résistance totale aux virus.

L'efficacité du polyribozyme de l'invention est surprenante au vu des résultats médiocres obtenus dans l'art antérieur avec les séquences antisens du gène de la protéine de capside, chacune de ces techniques impliquant une inactivation de l'ARN correspondant.

Une telle efficacité ne peut non plus être déduite des résultats présentés par Chen et al (Nucleic Acids Research 1992, 20(17): 4581-4589) concernant un polyribozyme spécifiquement dirigé contre l'ARN env de HIV-1. En effet, la glycoprotéine d'enveloppe du virus HIV-1 est une lipoprotéine membranaire, formant la couche la plus externe du virion chez certains virus et qui est une protéine différente des protéines de capside. En outre, plusieurs auteurs avaient déconseillé l'utilisation de polyribozymes agissant en trans parce que les ribozymes ne pouvaient pas fonctionner indépendamment les uns des autres et parce que les régions catalytiques ayant des séquences identiques avaient parfois tendance à s'hybrider entre elles, ce qui conduisait à des structures inactives (voir par exemple, Taira, HFSP Workshop "RNA-Editing - Plant Mitochondria", Abstract Book, Berlin, September 15-20, 1992). Les résultats obtenus selon l'invention sont inattendus compte tenu, d'une part, de la cible choisie, c'est-à-dire la protéine de capside et, d'autre part, de la méthode utilisée pour inactiver la cible, c'est-à-dire un polyribozyme.

Outre l'efficacité de l'inactivation, les polyribozymes de l'invention présentent un certain nombre d'avantages par rapport aux techniques connues :

- Les ribozymes fonctionnent comme des enzymes, catalysent spécifiquement le clivage de plusieurs ARN viraux sans modification de structure. Ce clivage enzymatique conduit à la destruction de tous les ARN viraux alors que l'expression de la protéine de capside qui inhibe l'infection virale fonctionne comme un inhibiteur de la multiplication du virus.
- Les ribozymes sont des molécules d'ARN non codantes qui ne peuvent induire des hétéroencapsidations ou générer de nouvelles souches virales.
- Alors que la spécificité de la tolérance induite par la protéine de capside est difficilement prévisible, les ribozymes peuvent être construits pour cliver spécifiquement une ou plusieurs souches virales, ou plusieurs virus apparentés si les bras complémentaires correspondent aux zones d'homologies conservées entre les différentes souches ou entre les différents virus apparentés.

Pour avoir une compréhension complète de l'invention, il est utile de préciser quelques informations concernant les ribozymes en général. Un ribozyme est une molécule d'ARN qui, en raison de sa séquence et de sa structure secondaire, possède une activité d'endoribonucléase lui permettant, lorsqu'il s'hybride avec une deuxième molécule d'ARN complémentaire, de cliver ce deuxième ARN. Ce dernier est donc un "substrat" pour le ribozyme.

Le ribozyme a deux parties essentielles :

(i) une séquence, qui sera appelée dans ce qui suit "séquence complémentaire", et qui est choisie de manière à ce qu'elle soit complémentaire du substrat que l'on veut cliver, ce qui permet l'hybridation des deux molécules ;
(ii) et une région catalytique qui a une séquence conservée indépendemment du substrat choisi et qui ne participe pas à l'hybridation avec le substrat en raison de sa structure secondaire en forme de "boucle".

Normalement, la région catalytique est située au sein de la séquence complémentaire, une partie de la séquence complémentaire se trouvant donc en 5' de la région catalytique, et l'autre partie en 3'. Ces fragments de séquence complémentaire de chaque côté de la région catalytique sont souvent appelés "bras hybridants".

L'objet de la présente invention est un polyribozyme. Plus particulièrement, il s'agit d'une séquence d'acide nucléique, dite "polyribozyme", ayant une activité d'endoribonucléase et étant capable d'inactiver le gène de la protéine de capside d'un virus, caractérisée en ce qu'elle comprend :

i) une séquence complémentaire d'une partie, au moins, du gène ou de son transcrit ou de ses intermédiaires de réplication et, incluses à des endroits distincts de cette séquence complémentaire :
ii) une pluralité de régions catalytiques provenant de ribozymes ;
iii) et, éventuellement, une ou plusieurs séquences non complémentaires du transcrit dudit gène, ladite ou lesdites séquences non complémentaires étant insérées entre 2 bases consécutives de la séquence complémentaire.

Le terme "polyribozyme" dans le contexte de la présente invention signifie une molécule d'ARN constituée d'un enchaînement, en tête-à-queue, de ribozymes, le ribozyme étant donc le motif unitaire du polyribozyme. En d'autres termes, il s'agit d'une série de régions catalytiques reliées entre elles par des bras hybridants, l'ensemble de ces bras constituant la séquence complémentaire. Le polyribozyme agit normalement comme une "uni-molécule" contre un seul transcrit, c'est-à-dire que les sites de clivage de chacune des régions catalytiques se trouvent sur un même transcrit : la protéine de capside. Le polyribozyme de l'invention peut également comprendre, outre les 2 parties essentielles [(i), séquence complémentaire] et [(ii), régions catalytiques] décrites ci-dessus, une ou plusieurs séquences (iii) non complémentaires du substrat. La nature et la fonction de ces séquences non complémentaires seront décrites en détail plus loin.

Parmi les 2 parties essentielles du polyribozyme, la séquence complémentaire est celle qui détermine le substrat. Dans le cas de la présente invention, il s'agit d'une séquence complémentaire du gène de la protéine de capside d'un virus, ou d'un fragment de ce gène. Lorsqu'il s'agit d'un virus à ARN(+), dont les gènes servent directement de mARN, la séquence complémentaire est réellement complémentaire du gène. Dans d'autres cas, elle est complémentaire du transcrit du gène. Elle peut également être complémentaire d'un intermédiaire de réplication.

La séquence complémentaire peut s'hybrider avec la totalité du gène de capside. Dans ce cas, la longueur totale de la séquence complémentaire varie en fonction de la longueur du gène de capside en question. En revanche, la séquence complémentaire peut s'hybrider seulement avec un fragment du gène. Le fragment en question doit être suffisamment long pour pouvoir inclure, dans la séquence correspondante du polyribozyme, au moins deux régions catalytiques. De manière générale, la longueur de la séquence complémentaire, sans compter les régions catalytiques (c'est-à-dire la somme des bras hybridants) peut varier d'environ 40 à 2000 bases. Une longueur de 400 a 1000 est préférée, de nombreux virus ayant un gène de la protéine de capside d'environ 1000 bases (par exemple, CMV, PLRV).

Le terme "complémentaire" dans le contexte de l'invention signifie un degré de complémentarité suffisamment élevé pour permettre l'hybridation stable entre le polyribozyme et ce substrat, et le clivage efficace du substrat. Lorsque le polyribozyme ne contient pas de séquence du type (iii), c'est-à-dire "non complémentaire", le degré de complémentarité est normalement de 100 %. La présence d'un certain nombre de mésappariements, par exemple jusqu'à 10 %, dans la séquence peut être tolérée à condition que cela n'empêche pas l'hybridation et le clivage du substrat.

La partie (ii) du polyribozyme, c'est-à-dire la région catalytique, provient de n'importe quel type de ribozyme approprié, par exemple "Tête de Marteau", "Hairpin", ou "Group I intron". Un même polyribozyme peut contenir des régions catalytiques provenant de différents types de ribozymes, par exemple "Tête de Marteau" et "Epingle à cheveux". De préférence, il s'agit de régions catalytiques provenant de ribozymes du type "Tête de Marteau" (Hammerhead) et dont la structure consensus est illustrée dans les figures 1A, B, C et D. Ces ribozymes sont décrits en détail dans la demande de brevet EP-A-321021 et WO-A-9119789.

Bien que les régions catalytiques illustrées dans la figure 1 aient une structure et une séquence conservées, il a été constaté que certains nucléotides peuvent être délétés, insérés, substitués ou modifiés sans nuire à l'activité du ribozyme. L'invention comprend l'utilisation, dans le polyribozyme, de ces régions catalytiques modifiées à condition que leur activité catalytique soit conservée. Cette activité peut être vérifiée en appliquant les tests décrits plus loin.

Par exemple, un ou plusieurs nucléotides de la région catalytique II illustrée dans la figure 1A peuvent être remplacés par des nucléotides contenant des bases telles que l'adénine, la guanine, la cytosine, la méthyl cytosine, l'uracile, la thymine, la xanthine, l'hypoxanthine, l'inosine ou d'autres bases méthylées. Les bases "conservées" C-G qui, ensemble, forment la première paire appariée de la boucle catalytique, peuvent être remplacées par U-A (Koizumi et al, FEBS Letts. 228, 2, 228-230, 1988).

Les nucléotides de la région catalytique illustrée dans la figure 1 peuvent aussi être modifiés chimiquement. Les nucléotides sont composés d'une base, d'un glucide et d'un groupement monophosphate. Chacun de ces groupements peut donc être modifié. De telles modifications sont décrites dans "Principles of Nucleic Acid Structure (Ed. Wolfram Sanger, Springer Verlag, New York, 1984). Par exemple, les bases peuvent porter des substituants tels que des groupements halogènes, hydroxy, amine, alkyl, azido, nitro, phényl, etc. La partie glucide des nucléotide peut aussi subir des modifications telles que le remplacement des groupements hydroxyl secondaires par des groupements halo, amino ou azido ou encore la 2'méthylation.

Le groupement phosphate des nucléotides peut être modifié par le remplacement d'un oxygène par N, S ou C donnant lieu respectivement à un phosphoramidate, phosphorothioate et phosphonate. Ces derniers peuvent présenter des propriétés pharmacokinétiques intéressantes.

Les bases et/ou les nucléotides de la région catalytique peuvent aussi porter des substituants tels que des acides aminés, par exemple la tyrosine ou l'histidine.

Il a également été constaté que des nucléotides supplémentaires pouvaient être insérés à certains endroits de la région catalytique sans nuire à l'activité du ribozyme. Par exemple, une base supplémentaire choisie parmie A, G, C ou U peut être insérée après $A^1$ dans la figure 1A ou 1B.

Selon une variante de l'invention, le polyribozyme peut comprendre, comme région catalytique, une ou plusieurs structures telles qu'illustrées dans la figure 1D. Cette structure, appelée "minizyme", est décrite dans la demande de brevet international WO-A-9119789. Elle représente une région catalytique du type "Tête de Marteau" dont la "boucle" a été remplacée par un groupe "P". P peut être une liaison covalente entre G et $^1A$, un ou plusieurs nucléotides (RNA ou DNA, ou un mélange, ou encore des dérivés décrits ci-dessus) ou tout atome ou groupement d'atomes autre qu'un nucléotide, qui n'affecte pas l'activité catalytique. Lorsque P représente une pluralité de nucléotides, elle peut contenir des appariements internes. La séquence et le nombre de nucléotides constituant le groupe "P" n'est pas critique et peut aller de 1 à 20 nucléotides par exemple, et de préférence de 1 à 6. Il est préférable de choisir une séquence dépourvue d'appariements internes du type Watson-Crick.

L'activité catalytique des polyribozymes de l'invention peut être vérifiée in vitro par la mise en contact du polyribozyme, ou d'une séquence qui, après transcription, donnera lieu au polyribozyme, avec le substrat suivie de la mise en évidence du clivage. Les conditions opératoires pour la réaction de clivage in vitro sont les suivantes : une température comprise entre 4 et 60° C, de préférence entre 20 et 55°C, un pH compris entre 7.0 et 9.0 environ, en présence de métaux divalents, tels que $Mg^{2+}$, à une concentration de 1 à 100 mM (de préférence 1 à 20 mm). Le polyribozyme est en général présent dans une proportion équimolaire avec le substrat, ou en excès. Les réactions de clivage in vitro se déroulent avantageusement selon le protocole décrit par Lamb et Hay (J. Gen. Virol. 1990, 71, 2257-2264). Cet article décrit également les conditions appropriées pour la transcription in vitro pour la production de ribozymes à partir d'oligodéoxyribonucléotides insérés dans des plasmides.

In vivo, les conditions de clivage sont celles existant naturellement dans la cellule.

Les ribozymes "Tête de Marteau" clivent le substrat immédiatement en aval d'un site "cible" XXX, de préférence XUX, dans lequel X représente l'une des 4 bases A, C, G, U et U représentent l'uracyl. Une séquence cible particulièrement préférée est XUY dans laquelle Y représente A, C ou U et X est souvent G, par exemple GUC. D'autres sites cibles sont possibles, mais moins efficaces, par exemple CAC, UAC, et AAC. Pour les ribozymes du type "Epingle à cheveux", une séquence cible préférée est AGUC.

Ces séquences cibles sont importantes dans la construction et le fonctionnement des polyribozymes, non seulement parce qu'elles indiquent les positions de clivage du substrat, mais aussi parce qu'ils déterminent la position à laquelle la région catalytique doit être insérée dans la séquence complémentaire. En effet, chaque région catalytique du polyribozyme doit se trouver à un endroit, dans la séquence complémentaire, qui correspond à un site XUX du transcrit. Par exemple, si un site XUX se trouve à la position 108 du gène de la protéine de capside et un autre à la position 205, une région catalytique est insérée à la position correspondant à 108 dans la séquence complémentaire et une autre à la 205.

Le motif XUX est un motif qui se présente très fréquemment dans les séquences d'ARN. Par exemple, il existe en moyenne un motif GUC toutes les 64 bases dans une séquence ayant un répartition de bases aléatoire et égale. Ceci signifie que le substrat contient normalement une pluralité de sites de clivage XUX. Comme indiqué ci-dessus, les régions catalytiques du polyribozyme se situent à des positions de la séquence complémentaire qui correspondent aux sites XUX. Ceci étant, il n'est pas nécessaire, pour obtenir un clivage efficace selon l'invention, d'inclure une région catalytique pour chaque séquence cible XUX du substrat. Selon l'invention, un clivage efficace est obtenu lorsque le polyribozyme contient au moins 2 régions catalytiques. Le nombre total de régions catalytiques incluses dans la séquence complémentaire est égal ou inférieur au nombre total de sites XUX présents dans le gène. Le polyribozyme de l'invention peut donc contenir un nombre très variable de régions catalytiques. Par exemple, pour le CMV, le polyribozyme peut contenir de 2 à 11 ou 12 régions catalytiques environ, lorsque la séquence cible est GUC. Dans le cas où il est décidé d'inclure dans la séquence complémentaire, un nombre de régions catalytiques inférieur au nombre de sites XUX dans le substrat, le choix des sites retenus peut se faire en respectant les critères suivants :

a) la distance entre 2 sites XUX ciblés et, par conséquent, entre 2 régions catalytiques dans le polyribozyme, doit être suffisamment longue pour permettre aux bras hybridants du polyribozyme situés entre les régions catalytiques correspondantes, de s'hybrider avec le substrat de manière stable et pour éviter que les régions catalytiques ne s'hybrident entre elles. Une distance d'au moins 8 bases, et de préférence d'au moins 14 bases, par exemple 20 bases environ est particulièrement avantageuse. Bien entendu, ce critère ne doit être pris en considération que lorsque le substrat contient des sites XUX très rapprochés. Autrement, si les sites XUX du substrat sont éloignés les uns des autres de plus de 8 à 20 bases, ce critère de choix n'a pas d'importance.

b) les sites XUX ciblés se trouvent de préférence dans une partie du gène de la protéine de capside qui n'a pas de structure secondaire importante. Ceci facilite l'accès du polyribozyme au substrat et augmente son efficacité.

c) les sites XUX ciblés peuvent faire partie des zones d'homologie conservée entre les différentes souches d'un même virus, ou entre différents virus apparentés. Les polyribozymes construits en respectant ce critère peuvent cliver spécifiquement plusieurs souches virales ou plusieurs virus apparentés. Par exemple, la région centrale du gène de la protéine de capside du PLRV est hautement conservée par rapport aux séquences des protéines de capside des virus apparentés BWYV et BYDV. Les sites XUX, et particulièrement GUX au sein de cette région centrale constituent donc des sites préférés pour un polyribozyme selon cette variante de l'invention.
Egalement à titre d'exemple, l'extrémité 5' (sur environ 100 bases) de la séquence de la protéine de capside du CMV est hautement conservée entre les souches I17F, FNY, M, I, O, Y, D et C. Au sein de cette séquence conservée, à la position 84, il existe un site GUC conservé dans toutes ces souches.
Selon cette variante de l'invention, un polyribozyme capable d'inactiver plusieurs souches du CMV comprend, parmi ses régions catalytiques, une région catalytique qui se trouve à l'endroit de la séquence complémentaire correspondant à la position 84 (voir exemples ci-dessous).

d) un autre critère de choix des sites XUX ciblés est l'absence d'homologie avec des gènes endogènes de la plante à transformer. En effet, bien qu'ils soient rares, certains virus possèdent des séquences qui trouvent un homologue dans le génome de plantes. Il est important donc d'éviter des sites XUX se trouvant au sein d'une telle séquence.

Selon un mode de réalisation particulièrement préféré de l'invention, le polyribozyme peut comprendre, outre les 2 parties essentielles (i) et (ii) décrites ci-dessus, un 3ème constituant (iii) qui est une ou plusieurs séquences non complémentaires du gène de protéine de capside du virus. Comme les régions catalytiques, ces séquences non complémentaires sont insérées à des endroits distincts de la séquence complémentaire, la complémentarité étant interrompue par l'insertion. De manière surprenante, il a été constaté par les inventeurs que la présence de telles séquences non complémentaires au sein des bras hybridants du polyribozyme n'empêche pas l'hybridation du polyribozyme avec le substrat, et dans certains cas, pouvait même améliorer l'efficacité de la réaction de clivage.

Ces séquences non complémentaires sont insérées entre 2 bases consécutives de la séquence complémentaire, la séquence non complémentaire formant ainsi une insertion colinéaire avec la séquence complémentaire. Dans ce cas, le polyribozyme a la structure :

$$((\text{bras hybridant - région catalytique - bras hybridant}) - (\text{séquence non complémentaire})_n)_p$$

dans laquelle n = 0 ou 1, et p > 1.

Comme exemple de ce mode de réalisation de l'invention, on peut citer un polyribozyme composé d'un enchaînement de ribozymes, dont les bras hybridants sont complémentaires à des fragments distincts, consécutifs et contigus du substrat et qui sont reliés entre eux par des séquences non complémentaires. En d'autres termes, dans une telle structure, l'ensemble des bras hybridants reconstituent la séquence complémentaire du gène de la protéine de capside.

La présence de séquences non complémentaires dans le polyribozyme signifie que la distance entre deux régions catalytiques du polyribozyme est plus grande que la distance entre les deux sites GUC correspondants dans le substrat. Selon cette variante de l'invention, la longueur des bras hybridants se trouvant de chaque côté d'une région catalytique doit être d'au moins 4 bases, de préférence d'au moins 8 bases de chaque côté et peut aller jusqu'à 800 à 1000 bases.

La nature de la ou des séquences non complémentaires peut être très variable selon sa fonction. Il peut s'agir de séquences ayant une fonction de "remplissage", c'est-à-dire servant à augmenter la distance entre deux régions catalytiques du polyribozyme, lorsque les deux sites XUX correspondants du substrat sont relativement proches l'un de l'autre. De cette façon, la formation de duplex inactifs entre deux régions catalytiques voisines peut être évitée. Il est également possible d'utiliser, comme séquences non complémentaires, des séquences ayant une structure secondaire déterminée, ce qui peut avoir pour effet d'empêcher un polyribozyme de longueur importante, par exemple de plus de 800 bases, de se replier sur lui-même dans une structure secondaire inactive. Comme exemple de ce type de structure, on peut citer la région catalytique d'un ribozyme rendu inactive par la délétion d'une ou de plusieurs bases essentielles. Ce mode de réalisation de l'invention est exemplifié par le polyribozyme 136 décrit dans les exemples ci-dessous.

La séquence non complémentaire du polyribozyme peut aussi avoir une fonction précise, par exemple, elle peut être constituée d'une séquence codante qui sert dans la sélection des transformants ou encore une séquence contenant un ribozyme agissant sur un substrat autre que la protéine de capside ou agissant en cis sur une partie du polyribozyme. De manière générale, la séquence non complémentaire ne code pas pour une protéine. Elle peut également contenir des multisites de clonage. La séquence non complémentaire a normalement une longueur comprise entre 2 et 500 bases, par exemple 20 à 100 bases. Lorsqu'il y a une pluralité de séquences complémentaires, elles peuvent constituer ensemble jusqu'à 90 % environ de la longueur du polyribozyme, par exemple 50 %.

Le polyribozyme de l'invention est normalement constitué d'ARN. Il est possible, néanmoins, de remplacer, par de l'ADN, certaines parties du polyribozyme, par exemple les bras hybridants ou des parties de ces bras, ou encore une partie de la région catalytique, en particulier la "boucle", à condition que l'activité catalytique soit maintenue (voir, par exemple, la substitution de l'ARN par de l'ADN décrite dans la demande de brevet international WO-9119789).

Le polyribozyme de l'invention peut être construit pour inactiver n'importe quelle protéine de capside virale. La protéine de capside est une sous-unité protéique codée par le génome viral et destinée à être incorporée dans la structure polymérique de la capside. En effet, la capside est constituée par la succession de sous-unités protéiques, identiques entre elles, qui s'accrochent le long de l'acide nucléique. Le groupement des sous-unités capsidiques peut entrainer une architecture hélicoïdale ou icosaédrique selon les virus. L'invention vise des polyribozymes dirigés contre les protéines de capsides de virus soit à particules hélicoïdales, soit à particules icosaédriques ainsi que de virus à enveloppe. L'enveloppe est une membrane lipidoprotéique entourant la nucléocapside.

Comme exemple de virus approprié, on peut citer un virus choisi parmi les groupes suivants : les Caulimovirus, par exemple le virus de la Mosaïque du Chou-Fleur (CaMV) ; les Geminivirus, par exemple le virus du Streak du Mais (NSV) ; les Reoviridae, par exemple le virus des Tumeurs de Blessures (WTV) ; les Rhabdoviridae, par exemple le Potato Yellow Dwarf Virus (PYDV), le Tomato Spotted Wilt Virus (TSWV), le virus de la Maladie Bronzée de la Tomate ; les Tobamovirus, par exemple le virus de la Mosaïque du Tabac (TMV) ; les Potexvirus, par exemple le virus X de la Pomme de Terre (PVX) ; les Potyvirus, par exemple le virus Y de la Pomme de Terre (PVY) ; les Carlavirus, par exemple le virus Latent de l'Oeillet (CLV) ; les Closterovirus, par exemple le virus de la J'aunisse de la Betterave (BYV) ; les Tobravirus, par exemple le virus du Rattle du Tabac (TRV) ; les Hordeivirus, par exemple le Virus de la Mosaïque Striée de l'Orge ; les Tymovirus, par exemple le virus de la Mosaïque Jaune du Navet (TYMV) ; les Lutéovirus, par exemple le virus de la Jaunisse Nanisante de l'Orge (BYDV) ou le virus de l'Enroulement de la Pomme de Terre (PLRV) ; les Tombusvirus, par exemple le Tomato Bushy Stunt Virus (TBSV) ; les Sobemovirus, par exemple le Southern Bean Mosaïc Virus (SBMV) ; le Virus de la Nécrose du Tabac (TNV) ; les Népovirus, par exemple le Virus des Taches en anneaux du tabac (TRSV) ; les Comovirus, par exemple, le Virus de la Mosaïque du Vigna (CPMV) ; le Virus de la Mosaïque Enation du Pois (PEMV) ; les Cucumovirus, par exemple le Virus de la Mosaïque du Concombre (CMV) ; les Bromovirus, par exemple le Virus de la Mosaïque du Brome (BMV) ; les Ilarvirus, par exemple le Virus du Streak du Tabac (TSV). Les séquences de ces protéines sont connues (voir par exemple, les nombreuses références bibliographiques citées dans l'oeuvre : "Eléments de Virologie Végétale", Pierre Cornuet, I.N.R.A. Paris, 1987, ISBN : 2-85340-808-6) .

Selon une variante particulièrement préférée, la protéine de capside est celle du Virus de la Mosaïque du Concombre (CMV). Le Virus de la Mosaïque du Concombre est un virus appartenant au groupe des Cucumovirus d'une grande importance agronomique car plus de 750 espèces de plantes peuvent être infectées par le CMV. Le CMV est un virus à plusieurs composants formé de particules icosaédriques renfermant trois ARN génomiques (ARN 1 à 3) et un ARN subgénomique (ARN 4). L'ARN 3 contient une copie du gène de la protéine de capside, cependant l'ARN4 subgénomique qui dérive de l'ARN3 sert de matrice pour la synthèse de la protéine de capside. Les différentes souches de CMV se répartissent en deux groupes :

* le sous-groupe I qui comprend les souches C, D, FNY, y, I17F et Chi ;
* le sous-groupe II auxquels appartiennent les souches Q et WL.

La comparaison des séquences en acides aminés des protéines de capside du CMV entre les souches d'un même sous-groupe montre une homologie de 95 %. Entre les sous-groupes I et II, les homologies de séquence sont moins importantes, de l'ordre de 80 %.

Les polyribozymes de l'invention dirigés contre la protéine de capside du CMV (souche I17F) se sont révélés extrêmement efficaces dans l'inactivation des différentes souches du virus CMV, et ont abouti à une résistance totale des plantes transformées.

Outre les polyribozymes, l'invention vise également un procédé pour rendre une plante résistante à un virus, caractérisé par l'introduction dans la plante d'un polyribozyme ou d'une séquence codant pour un polyribozyme tel que décrit ci-dessus.

Normalement, l'introduction du polyribozyme dans la plante s'effectue par transformation génétique, une séquence d'ADN codant pour le polyribozyme étant donc intégrée de manière stable dans le génome de la plante.

Tous les moyens connus pour introduire de l'ADN étranger dans des plantes peuvent être utilisés, par exemple Agrobacterium, électroporation, fusion de protoplastes, bombardement avec canon à particules, ou pénétration d'ADN dans des cellules comme le pollen, le microspore, la graine et l'embryon immature, vecteurs viraux tels que les Geminivirus ou les virus satellites. Agrobacterium tumefaciens et rhizogenes constituent le moyen préféré. Dans ce cas, la séquence codant pour le polyribozyme est introduite dans un vecteur approprié avec toutes les séquences régulatrices nécessaires telles que promoteurs, terminateurs, etc... ainsi que toute séquence nécessaire pour sélectionner les transformants.

L'invention vise également les plantes transgéniques obtenues par le procédé. Plus particulièrement, elle vise des plantes transgéniques résistantes à un virus, caractérisées en ce qu'elles contiennent, dans leur génome, une séquence qui donne lieu, après la transcription, à un polyribozyme selon l'invention.

Dans le contexte de l'invention, les termes "résistance totale" signifient qu'il y a absence totale de symptômes ; "tolérance" signifie que la plante est infectée, c'est-à-dire qu'elle manifeste des symptômes, mais qu'ensuite, elle récupère. Le terme "sensible" signifie que la plante présente des symptomes et réplique le virus. L'expression "Type résistance" correspond à la somme des plantes totalement résistantes et des plantes tolérantes.

La nature "résistante" des plantes transgéniques de l'invention peut être testée de la manière suivante : une autofécondation, ou un croisement avec un génotype non transformé, est effectuée sur un descendant primaire pour obtenir $T_1$. Ensuite, $T_1$ est inoculé avec le virus en question. Selon l'invention, après autofécondation, 75 % des plantes $T_1$ sont totalement résistantes. Dans le cas d'un croisement avec un génotype non transformé, 50 % des plantes sont totalement résistantes (ces chiffres sont obtenus, selon l'invention, en testant une population entière de plantes ayant fait l'objet d'un procédé de transformation et de régénération. Il est à noter que seulement 75 % de ces plantes sont transformées).

La nature transformée d'une plante peut être vérifiée en effectuant une analyse "Southern" et l'expression de la séquence introduite par la transformation est vérifiée en effectuant une analyse "Northern". Ces analyses sont décrites dans les exemples suivants.

Les méthodes de transformation et de régénération de plantes connues dans l'art se prêtent parfaitement à la production de plantes transgéniques protégées par le polyribozyme de l'invention. Comme exemple, on peut citer la méthode de transformation et de régénération du melon décrit dans la demande de brevet n° EP-A-0412912.

Les plantes transgéniques résistantes au CMV sont particulièrement préférées, par exemple le melon, le concombre, la courgette, la tomate, le poivron, le haricot.

Différents aspects de l'invention sont illustrés dans les figures :

- la figure 1 montre les structures préférées des régions catalytiques du polyribozyme de l'invention, ces régions étant entourées, de chaque côté, d'une séquence complémentaire d'une partie de la protéine de capside d'un virus :

  (i) dans la figure 1A : X représente A, G, C ou U ; chaque X étant identique ou différent ; $n + n' \geq 6$, n et n' étant identiques ou différents ; (*) représente une liaison d'hydrogène entre ribonucléotides complémentaires ; X' et X" représentent des oligoribonucléotides qui sont complémentaires l'un de l'autre sur au moins une partie de leur longueur et qui peuvent éventuellement être reliés l'un à l'autre par au moins un nucléotide, ce qui forme ainsi une boucle. Un nucléotide supplémentaire choisi parmi A, G, C ou U peut être inséré après $A^1$. La région catalytique du ribozyme est représentée par la partie (II) de la figure 1A, et les bras hybridants par la partie (I).
  (ii) dans la figure 1B : X, (*), n, n' et $A^1$ ont la même signification que dans la figure 1A. M et $M' \geq 1$ et sont identiques ou différents. B représente une liaison, une paire de bases, un ribonucléotide ou un oligoribonucléotide contenant au moins 2 ribonucléotides.
  (iii) la figure 1C représente un modèle préféré de ribozymes (Haseloff et Gerlach, 1988). L'ARN substrat peut avoir toute séquence (X) autour du site de clivage GUC complémentaire du ribozyme. La flèche indique le site de clivage. Les bases conservées apparaissent en noir.
  (iv) la figure 1D représente la structure d'un ribozyme (dit "minizyme") dont la boucle de la région catalytique est remplacée par un élément "P". P peut être 1 nucléotide au moins (ribonucléotides, désoxyribonucléotides, dérivés ou un mélange), à condition que, lorsque les séquences $(X')_n$ et $(X)_{n'}$ et P sont constituées exclusivement de ribonucléotides, les ribonucléotides du groupe "P" ne soient pas appariés entre eux par des liaisons "Watson et Crick". "P" peut aussi être une liaison ou tout atome ou groupement d'atomes qui n'affecte pas l'activité catalytique du ribozyme. X a la même signification que dans la figure 1A.

- la figure 2 montre la séquence de la protéine de capside du CMV (I17F), les sites GUC étant soulignés.

- la figure 3 présente les structures des oligodéoxyribonucléotides A, B, C, E utilisés pour les expériences de mutagénèse dirigée dans le but d'introduire le site catalytique du TobRSV en différents endroits de la séquence de la protéine de capside du CMV (représentés en hybridation avec la séquence d'ADN matrice).

- la figure 4 illustre la structure des gènes construits pour induite une résistance au CMV :

  (i) protéine de capside ;
  (ii) polyribozyme 136 : séquence complémentaire de la protéine de capside portant 2 ribozymes ;
  (iii) polyribozyme 161 : séquence complémentaire de la protéine de capside portant 3 ribozymes ;
  (iv) polyribozyme 163 : fragment complémentaire de la protéine de capside portant 3 ribozymes ;
  (v) polyribozyme 165 : séquence complémentaire de la protéine de capside portant 4 ribozymes.

- la figure 5 montre l'analyse Northern des plantes de melon transgéniques (transformants primaires, descendants T1 et T2 dans certains cas) exprimant le polyribozyme 136 :

  T0 : transformant primaire ;

T1 : descendant T1 ;
T2 : descendant T2 ;
A : lignée 141.1 ;
NT : témoin non transformé.

- la figure 6 montre l'analyse Southern des plantes de melon transgéniques (transformants primaires, descendants T1 et T2 dans certains cas) exprimant le polyribozyme 136 :

T0 : transformant primaire ;
T1 : descendant T1 ;
T2 : descendant T2 ;
NT : témoin non transformé.

- la figure 7 montre l'analyse Southern des plantes de melon transgéniques (transformants primaires, descendants T1 et T2 dans certains cas) exprimant le gène de la protéine de capside :

T0 : transformant primaire ;
T1 : descendant T1 ;
T2 : descendant T2 ;
A : lignée 88.105 ;

B : lignée 159.8 ;
C : lignée 164.23 ;
NT : témoin non transformé.

- la figure 8 montre l'analyse Western des plantes de melon transgéniques (transformants primaires) transformées avec pBIOS135 :

| | |
|---|---|
| A, B, C, D et E : | transformants primaires de 5 lignées ; |
| NT : | témoin non transformé ; |
| R : | reconstruction avec 20 ng de CMV. |

- la figure 9 montre le développement des symptômes du CMV au cours du temps dans des lignées transformées avec pBIOS 135 :
J : jour ;
■ : plantes sans symptômes ;
▒ : plantes tolérantes ;
: plantes sensibles.

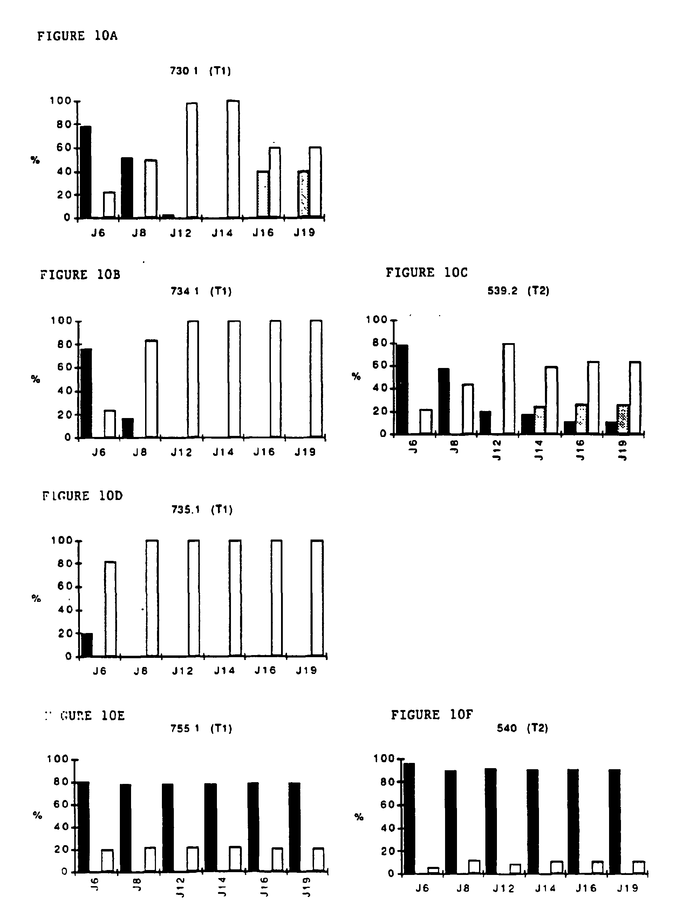

- la figure 10 montre le développement des symptômes du CMV au cours du temps dans des lignées transformées avec pBIOS 136 :
J : jour ;
■ : plantes sans symptômes ;
▒ : plantes tolérantes ;
: plantes sensibles.

EXEMPLES

Les exemples suivants décrivent la construction de 4 polyribozymes contenant 3 ou 4 ribozymes composés de la structure consensus Hammerhead de 24 bases (Figure 1) et de bras complémentaires de la séquence de la protéine de capside du CMV (souche I17F) de tailles différentes. La numérotation de la séquence nucléotidique de la protéine de capside présentée dans ces exemples correspond à celles utilisées dans la demande de brevet EP-A-0412912.

Chacun de ces ribozymes coupe une séquence GUC différente le long de la séquence du gène de la protéine de capside du CMV. La structure de ces construits est illustrée dans la figure 4 :

- Le polyribozyme 136, long de 1074 bases, contient les ribozymes A (position 84) et B (position 108) et le ribozyme C* (position 204) dont un G et un A (positions 20 et 21) ont été délétés, les ribozymes étant entourés par des bras complémentaires longs de :

  - 82 nucléotides de l'extrémité 5' au ribozyme A ;
  - 22 nucléotides entre les ribozymes A et B ;
  - 94 nucléotides entre les ribozymes B et C* ;
  - et 803 nucléotides du ribozyme C* à l'extrémité 3'.

- Le polyribozyme 161, long de 1076 bases, est identique au polyribozyme 136 à la seule différence que le ribozyme C position 204 possède le G et le A délétés dans le ribozyme C*.
- Le polyribozyme 163, long de 426 nucléotides, contient les 3 ribozymes A (position 84), B (position 108) et C (position 204) entourés par des bras complémentaires longs de :

  - 82 nucléotides de l'extrémité 5' au ribozyme A ;
  - 22 nucléotides entre les ribozymes A et B ;
  - 94 nucléotides entre les ribozymes B et C ;
  - et 153 nucléotides du ribozyme C à l'extrémité 3'.

- Le polyribozyme 165, long de 1099 nucléotides, contient les 4 ribozymes A (position 84), B (position 108), C (position 204) et E (position 608) qui sont entourés par des bras complémentaires longs de :

  - 82 nucléotides de l'extrémité 5' au ribozyme A ;
  - 22 nucléotides entre les ribozymes A et B ;
  - 94 nucléotides entre les ribozymes C et E ;
  - 399 nucléotides du ribozyme E à l'extrémité 3'.

Les polyribozymes ne contiennent pas les signaux nécessaires à leur expression et à leur intégration dans le génome des plantes. Un promoteur constitutif ou non constitutif (par exemple un promoteur viral ou bactérien, tel que NOS, ou de plante, tel que celui de la Rubisco et celui de l'ubiquitine) doit être placé en 5' du polyribozyme et une séquence poly (A) doit être placée en 3'. Plusieurs promoteurs qui fonctionnent chez les plantes sont utilisables ; les inventeurs ont choisi le promoteur 35S dérivé du virus de la mosaïque du chou-fleur (CaMV) réputé comme le plus fort promoteur constitutif. Le signal de polyadénylation poly(A) peut être celui du gène 35S du CaMV, celui de gènes isolés de plantes, de l'octopine synthase ; les inventeurs ont choisi le signal poly (A) dérivé du gène de la nopaline synthase (tnos). Les cassettes d'expression construites ont été introduites dans le vecteur de transformation pBIOS 4 dérivant du vecteur pBI 121 contenant le gène de résistance à la kanamycine (gène néo codant pour la néomycine phosphotransférase) et le gène iud A codant pour la glucuronidase. Le protocole de transformation génétique de cotylédons de melon et de régénération de plantes transgéniques de melon est identique à celui décrit dans le brevet "melon transgénique" n° EP-A-0412912 au nom de BIOSEM.

**EXEMPLE 1 : CONSTRUCTION D'UN RIBOZYME DIRIGE CONTRE LE GENE DE LA PROTEINE DE CAPSIDE DU CUCUMBER MOSAIC VIRUS, SOUCHE I17F :**

Le phagémide bluescribe pBSIISK$^-$ (STRATAGENE) contenant de l'ADN complémentaire de l'ARN4 du CMV souche I17F appelé pBIOS 113, dont la procédure d'obtention est décrite dans la demande de brevet européen EP-A-0412912, a servi de point de départ à la construction des différents ribozymes utilisés pour obtenir des melons transgéniques résistants à différentes souches de CMV.

Le site catalytique du satellite TobRV (Tobacco Ringspot Virus) a été introduit à différentes positions de la séquence du gène de la protéine de capside. La séquence de l'ADN complémentaire à l'ARN 4 du CMV, souche I17F longue de 1007 bp est présente dans la figure 3 du EP-A-0412912 ; la partie codant pour la protéine de capside est présentée avec la séquence en acides aminés.

Comme le ribozyme de type "Hammerhead" décrit par Haselhoff et Gerlach coupe préférentiellement après le C des motifs GUC, 4 positions sur la séquence codante de la protéine de capside ont été retenues (Figure 2) :

* Position A, nucléotide 84,
* Position B, nucléotide 108,
* Position C, nucléotide 204,
* Position E, nucléotide 608.

Afin d'introduire les 24 bases du ribozyme communément appelé "hammerhead" (tête de marteau en français) à ces positions là, il a été décidé d'utiliser la méthode de mutagénèse sur ADN simple brin développée par KUNKEL (Proc. Nat. Acad. Sci 82 : 488-492) faisant appel à des oligodésoxyribonucléotides de synthèse qui hybrident partiellement avec l'ADN que l'on doit mutagénéiser et qui servent d'amorce à leur extrémité 3' pour une synthèse complémentaire à l'aide d'une DNA polymérase. Les quatre oligonucléotides suivants ont été commandés à la société EUROGENTEC :

Oligo A :
5' TCGACGGTTACCTGATGAGTCCGTGAGGACGAAACCAGCACTGGTTG 3'

Oligo B :
5' CGGGAACCACCTGATGAGTCCGTGAGGACGAAACGCGGACGACG 3'

Oligo C :
5' GTTAATAGTTGCTGATGAGTCCGTGAGGACGAAACGACCAGCTGC 3'

Oligo E :
5' GAATACACGAGCTGATGAGTCCGTGAGGACGAAACGGCGTACTTTC 3'

Grâce à ces 4 oligonucléotides et au kit de mutagénèse dirigée, acheté auprès de la société BIORAD (ref 170-3576), de l'ADN simple brin produit à partir du phagémide pBIOS 113 a été mutagénéisé. La figure 3 présente l'hybridation des 4 oligonucléotides sur l'ADN simple brin cible et les flèches matérialisent l'action de la DNA polymérase synthétisant le brin complémentaire. L'analyse des quelques clones recombinants obtenus après transformation d'E. coli a tout d'abord été faite par digestion avec des enzymes de restriction pour détecter un accroissement de taille de certains fragments. Un des clones, pBIOS 116, qui contenait apparemment 3 sites catalytiques a été séquencé, à l'aide du kit "sequenase R" version II de la société United States Biochemicals, en utilisant un oligonucléotide de 22 bases, oligo n°13 (position 53 à 74). Les résultats ont révélé l'insertion parfaite des sites catalytiques A et B et l'insertion imparfaite du site catalytique C. Une délétion des deux bases à la position 20 et 21 (G et A) du site catalytique a eu lieu. Bien que ce site ne puisse être fonctionnel pour le clivage, comme démontré par Lamb et Hay (Journal of General Virology, 1990, 71 : 2257-2264), il a été décidé de cloner le fragment d'ADN d'environ 1100 pb (1007 bp + 21 bp x 2 + 19 bp + séquences adjacentes en 5' et 3' du polylinker) en orientations inverses dans le vecteur d'expression pBIOS 3 (Perez et al, Plant. Mol. Biology 1989, 13 : 365-373), afin d'étudier l'effet de la présence de séquences non complémentaires dépourvues d'activité ribozymique, dans le polyribozyme. Pour cela, le fragment Kpn I - Xba I de pBIOS 113 a été cloné aux sites Kpn-Xba I du plasmide pGEH7 2f (+) obtenu auprès de la société PROMEGA BIOTECH ; ceci dans le but d'avoir des sites BamHI de part et d'autre de la séquence de la protéine de capside contenant les sites catalytiques. Le plasmide résultant pBIOS 151 a ensuite été digéré par BamHI et le fragment considéré (polyribozyme 136, figure 4) a été introduit dans le site BamHI de pBIOS 3. Un clone recombinant contenant le fragment en orientation antisens, sous le contrôle du promoteur constitutif fort 35S du virus de la Mosaïque du chou-fleur et du terminateur du gène de la nopaline synthétase, a été retenu et appelé pBIOS 125.

Le fragment EcoRI de ce plasmide contenant le ribozyme (fragment complémentaire avec deux sites catalytiques fonctionnels et un délété), sous le contrôle des séquences de régulation transcriptionnelle citées ci-dessus, a été clone au site EcoRI du vecteur binaire pBIOS 4. Ce dernier dérive du vecteur pBI 121 (Jefferson et al, 1987 ; EMBO Journal 6 : 3901-3907) qui a été modifié par la suppression du site EcoRI situé en 3' du gène codant pour la β-glucuronidase et la création d'un site EcoRI situé en 5' de ce même gène. Le vecteur binaire résultant, pBIOS 136, a servi aux différentes expériences de transformation après conjugaison triparentale dans la souche d'Agrobacterium tumefaciens désarmée RC58'3, qui dérive de la souche C58'3 (Mullineaux et al, Plant Science 63 : 237-245 1989) et est en fait un mutant spontané résistant à la rifampicine.

**EXEMPLE 2 : CONSTRUCTION DE DIFFERENTS RIBOZYXES DIRIGES CONTRE LE GENE DE LA PROTEINE DE CAPSIDE DU CUCUHBER MOSAIC VIRUS, SOUCHE I17F :**

Etant donné que le polyribozyme 136 (figure 4) décrit dans l'exemple 1 ne contenait pas le site catalytique dirigé contre la position 608 et que celui dirigé contre la position 204 était incomplet, de nouvelles expériences de mutagénèse dirigée ont été initiées. Pour cela, le fragment EcoRI du plasmide pBIOS 125 a été cloné dans le phage M 13 mp 18 digéré par EcoRI, obtenu auprès de la société PHARMACIA. Un phage recombinant permettant d'encapsider le brin codant du gène de la protéine de capside contenant les deux sites catalytiques corrects a pu être caractérisé et il a servi de matrice pour les nouvelles expériences de mutagénèse utilisant les oligodésoxyribonucléotides C et E (ensemble ou séparément). Ces dernières ont été conduites comme celle présentée dans l'exemple précédent, excepté que le rendement en matrice simple brin est beaucoup plus favorable car le matériau de départ était un phage. Différents clones recombinants ont été séquences en utilisant l'oligonucléotide n°13 et un oligonucléotide de 19 mers (position 694 à position 676), ce dernier permettant de séquencer le site catalytique introduit à la position 608. Les clones contenant des sites catalytiques conformes ont servis à la construction des vecteurs binaires pBIOS 161, pBIOS 163 et pBIOS 165 (voir figure 4). Le vecteur binaire pBIOS 161 est identique à pBIOS 136 excepté que pBIOS 161 contient le site catalytique C non délété.

Dans le cas de gènes codant pour les ribozymes hybridant avec la totalité de la séquence du gène de la protéine de capside et contenant 3 sites catalytiques fonctionnels ABC (cas de pBIOS 161) ou quatre sites catalytiques fonctionnels A, B, C, E (cas de pBIOS 165), le clonage au site EcoRI du vecteur binaire pBIOS 4 s'est effectué directement après purification du fragment EcoRI comprenant le promoteur 35S, le ribozyme, le terminateur NOS. Dans le cas du ribozyme contenant 3 sites catalytiques A, B, C et hybridant avec seulement les 360 premières bases de l'extrémité 5' de l'ARN 4 du CMV (cas de pBIOS 163), une délétion de la partie 3' restante a été faite après digestion avec l'enzyme de restriction HindIII (positions 361 dans la séquence du gène de la protéine de capside et du site HindIII situé à la bordure 3' de cette séquence et provenant d'un polylinker). Ensuite le fragment EcoRI du plasmide ainsi délété a été cloné au site EcoRI du vecteur binaire pBIOS 4.

<u>Transformation et régénération de melons exprimant les polyribozymes</u> :

Les 3 derniers vecteurs binaires ont été introduits dans <u>Agrobacterium</u> et utilisés en transformation comme décrit dans la demande de brevet EP-A-0412912.

**EXEMPLE 3 : ANALYSE MOLECULAIRE DES PLANTES TRANSGENIQUES :**

* <u>Analyses Northern</u> :

Les plantes de melons obtenues après transformation avec pBIOS 136 ont été analysées par Northern blot (figure 5). Les ARN totaux ont été extraits à partir de jeunes feuilles de plantes transgéniques et non transgéniques cultivées en serre, selon le protocole de <u>Chandler et al</u> (Plant Physiology (1983) <u>74</u> : 47-54).

Ils ont été soumis à une électrophorèse en gel d'agarose 1 % dénaturant, transférés sur Hybond C et hybridés à la sonde constituée des fragments résultant d'une triple digestion du fragment BamHI, qui correspond au fragment complet complémentaire du gène de la protéine de capside, porteur des 3 ribozymes dont deux fonctionnels (polyribozyme 136). La triple digestion favorise l'hybridation entre séquences homologues et permet d'obtenir un signal plus intense.

L'homogénéité des quantités d'ARN totaux déposés sur le gel et la qualité des ARNs ont été vérifiées par coloration de la membrane au bleu de méthylène. Les résultats obtenus au niveau transcriptionnel pour les transformants primaires, les descendances T1 et T2 correspondantes, sont illustrés par la figure 2 et montrent que :

- un transcrit majeur de taille 1,45 Kb est observé dans tous les échantillons provenant de plantes transformées et ne l'est pas dans le témoin négatif ;
- le nombre de transcrits varie considérablement selon les transformants primaires. Ceci peut s'expliquer par des intégrations du T-DNA ou de fragments de T-DNA dans des loci différents du génome végétal et donc par des effets environnementaux :

- aucune corrélation n'existe entre les niveaux transcriptionnels des transformants primaires et ceux de leurs descendances T1 et T2.

* <u>Analyses Southern :</u>

Les plantes de melons obtenues après transformation avec pBIOS 136 ou pBIOS 135 ont été analysées par Southern blot (figures 6 et 7). Les ADN totaux ont été extraits à partir de jeunes feuilles de plantes transgéniques (transformants primaires, descendants T1 et T2 dans certains cas) et non transgéniques cultivées en serre, selon le protocole de <u>Dellaporta et al</u> (Plant Molecular Biology Reporter (1983) <u>1</u> : 19-21). Ils ont été hydrolysés par EcoRI (site de clonage de la cassette d'expression du gène d'intérêt dans pBIOS4), soumis à l'électrophorèse en gel d'agarose 0,8 %, transférés sur Hybond N+ et hybridés avec les trois sondes, gène npt II, polyribozyme 136 triplement digéré (Cf analyse Northern) et gène gus.

Les résultats obtenus pour 5 événements de transformation avec pBIOS 136 (Figure 6) montrent que :

- **Lignées 146.42 :**
  Dans le cas du transformant primaire 146.42, de son descendant T1 et de deux descendants T2, les profils d'hybridation avec les 3 sondes sont identiques, ce qui n'indique aucune ségrégation des fragments du T-DNA et probablement un seul locus. Le polyribozyme 136 est présent en plusieurs copies dans le génome végétal. En effet, 4 bandes d'hybridation de taille 1,75 Kb ; 4,4 Kb ; 8,3 Kb et 8,8 Kb sont visibles. La bande de 1,75 Kb correspond à la bande de taille théorique attendue avec le couple (EcoRI, polyribozyme 136). La bande de 4,4 Kb s'hybride à la fois au polyribozyme 136 et au gène npt II. De plus, l'analyse avec le couple EcoRI/npt II ne permet pas de déceler cette bande, ce qui indique la présence d'une seule copie du gène npt II dans le génome végétal. Les bandes de 8,3 Kb et de 8,8 Kb s'hybrident au polyribozyme 136 et au gène gus. Seules ces deux bandes sont détectées par le couple EcoRI/gus, ce qui révèle la présence de deux copies du gène et/ou partie du gène gus. L'hybridation des 3 sondes aux ADN des plantes T0, T1 et T2 digérés par Xba I permet également de supposer la présence d'un seul locus.

- **Lignées 146.34 :**
  Dans le cas du transformant primaire 146.34 et de son descendant T1, seules certaines bandes d'hybridation du transformant primaire subsistent chez l'individu T1, ce qui souligne une élimination de certains fragments du T-DNA. Pour l'analyse du couple EcoRI/polyribozyme 136, 3 bandes de taille 1,75 Kb, 3 Kb et 5,3 Kb sont communes aux plantes T0 et T1 et 3 bandes supplémentaires de taille 7,3 Kb, 6,8 Kb et 4,25 Kb caractérisent la plante T0. Ceci indique la présence d'au moins 3 et 6 copies du polyribozyme 136 respectivement dans les plantes T1 et T0. En hybridation npt II, les deux bandes EcoRI de 3 Kb et 5,3 Kb sont détectées dans les plantes T0, ce qui montre la présence de deux copies du gène et/ou partie du gène npt II. Seule la bande EcoRI de 3 Kb est visible dans la plante T1, ce qui dénote la présence d'une seule copie du gène npt II. En hybridation gus, les 3 bandes EcoRI de taille 4,25 Kb ; 5,3 Kb et 6,8 Kb sont trouvées dans la plante T0 tandis que seule la bande de 5,3 Kb est présente dans le descendant T1. Ceci révèle la présence de 3 copies et d'une copie du gène et/ou partie du gène gus dans les plantes T0 et T1 respectivement. Par ailleurs, l'hybridation des 3 sondes aux ADNs des plantes T0 et T1 digérés par Xba I permet de supposer l'existence de 2 loci.
- **Lignées 146.30 et 146.28 :**
  Dans le cas des transformants primaires 146.30 et 146.28 ainsi que de leurs descendants T1 respectifs, des observations similaires sont réalisées.
- **Lignée 141.1 :**
  Dans le cas du transformant primaire 141.1 et de son descendant, les profils d'hybridation sont semblables. Des bandes EcoRI de 1,75 Kb ; 10 Kb et 10 Kb sont révélées avec les sondes polyribozyme 136, npt II et gus respectivement. Ceci montre la présence d'une seule copie du T-DNA intègre dans le génome végétal.

En conclusion, la stabilité génétique est observée dans le cas du transformant 146.42 et de sa descendance (T1 et T2) et dans celui du transformant 141.1 et de son descendant T1. Un nombre élevé de copies du T-DNA ou fragments du T-DNA caractérise 4 des lignées étudiées tandis que la lignée 141.1 ne possède qu'une copie intègre du T-DNA.

Par ailleurs, la présence d'une copie du T-DNA et/ou portion du T-DNA est également à noter chez les lignées transformées avec pBIOS 135 (Figure 7).

* <u>Analyses Western</u> (analyse comparative) :

Les plantes de melons transgéniques transformées avec pBIOS 4 contenant la cassette d'expression du gène de la protéine de capside ont été analysées par Western blot. La demande de brevet EP-A-0412912 du 11.08.1989 au nom de BIOSEM relate la méthodologie employée et les résultats obtenus. Cependant, il est à souligner que le taux d'expression du gène de la protéine de capside varie de 0,001 % et 0,4 % des protéines totales. La figure 8 illustre par quelques exemples cette variation du taux d'expression, qui dépend de l'âge de la feuille et probablement d'effets environnementaux du génome végétal.

**EXEMPLE 4 : TESTS DE RESISTANCE AU CMV DES MELONS TRANSFORMES AVEC LE POLYRIBOZYME 136 ET LA PROTEINE DE CAPSIDE (TEST COMPARATIF) :**

Les plantes de melon (génotype TEZIER 10) transgéniques exprimant le gène de la protéine de capside ou le polyribozyme 136 ont été autofécondées ou croisées avec des plantes du génotype TEZIER 10 non transformées. Les graines issues de ces autofécondations (génération T1) et d'autofécondations ultérieures (génération T2, etc) ont été semées en phytotron (enceinte climatisée avec photopériode de 16 h lumière).

Au stade 2 à 4 feuilles, 23 plantes issues de la descendance ont été inoculées mécaniquement avec un broyat de feuilles fralches infectées par du CMV souche TL28.

Après 16 jours d'infection (période optimale), les symptômes (mosaïque, gaufrage du limbe, jaunissement) ont été évalués. Les plantes infectées se classent en trois catégories :

- les plantes résistantes (R) qui n'ont aucun symptôme ;
- les plantes sensibles (S) qui ont des symptômes ;
- les plantes tolérantes (T), qui sont sont les plantes qui "récupèrent", c'est-à-dire que les feuilles naissantes se développent sans symptôme tandis que les feuilles âgées présentent des symptômes.

Plusieurs cycles de "récupération" peuvent avoir lieu. L'évolution des feuilles naissantes saines en feuilles infectées ou non dépend des conditions climatiques.

Les plantes de "type résistant" comprennent à la fois les plantes sans symptôme et les plantes tolérantes.

Les témoins utilisés dans le test de résistance au CMV sont :

- témoins sensibles : TEZIER 10 et Vedrantais ;
- témoins résistants possédant au moins trois gènes récessifs du CMV, Virgos et Free Cucumber.

* Résistance au CMV souche TL 28 de lignées de melon en génération T1 exprimant le gène de la protéine de capside :

Les résultats obtenus pour 20 lignées sont donnés dans le tableau 1 et montrent que :

- L'infection avec TL 28 conduit à l'obtention de 3 à 30 % de plantes T1 sans symptôme. Le témoin non transformé TEZIER 10, inoculé avec TL 28 présente 0 % de plantes sans symptômes. Les témoins résistants Virgos et Free Cucumber possèdent 92 et 100 % de plantes sans symptômes respectivement et ne présentent aucun phénomène de tolérance.
- Le phénomène de récupération existe à une forte proportion. Quatorze des lignées étudiées montrent un pourcentage non négligeable (22 à 59 %) de plantes T1 tolérantes.
- Le pourcentage de plantes T1 résistantes et tolérantes n'est pas corrélé au taux d'expression de la protéine de capside. En effet, les lignées 159.8 et 164.2, par exemple, qui ont un taux d'expression de la protéine de capside identique (0,01 %) présentent des niveaux de résistance différents. Les individus T1 de la lignée 159.8 sont tous sensibles tandis que ceux de la lignée 164.2 sont sans symptôme à 26 % et tolérants à 48 %.
- la plupart des lignées exprimant le gène de la protéine de capside ont été obtenues par autofécondation. La fréquence théorique attendue du gène de la protéine de capside est de 75 % dans les plantes T1 inoculées avec le CMV. Le pourcentage de plantes de type résistant (sans symptôme et tolérants) varient entre 25 et 82 % selon les lignées transgéniques.

**TABLEAU 1**

**RESISTANCE AU CMV SOUCHE TL28 DE PLANTES DE MELON EN GENERATION T1 EXPRIMANT LE GENE DE LA PROTEINE DE CAPSIDE**

| LIGNEES | CROISEMENT | % CP | % R | % T | % S |
|---|---|---|---|---|---|
| 88.105 | I | 0,01 | 3 | 22 | 75 |
| 131.6 | BC | 0,06 | 9 | 0 | 81 |
| 145.1 | I | | 4 | 0 | 96 |
| 145.2 | BC | 0,06 | 22 | 35 | 43 |
| 153.4 | I | 0,1 | 0 | 30 | 70 |
| 153.5 | I | 0,06 | 17 | 57 | 26 |
| 153.6 | I | 0,13 | 18 | 32 | 50 |
| 153.8 | I | 0,01 | 17 | 43 | 40 |
| 153.9 | I | 0,06 | 30 | 39 | 31 |
| 153.19 | I | 0,05 | 29 | 52 | 19 |
| 153.20 | I | 0,01 | 30 | 39 | 31 |
| 153.22 | I | 0,4 | 18 | 27 | 55 |
| 159.4 | BC | 0,002 | 0 | 0 | 100 |
| 159.8 | I | 0,01 | 0 | 0 | 100 |
| 164.2 | I | 0,01 | 26 | 48 | 26 |
| 164.23 | BC | 0.01 | 30 | 35 | 35 |
| 166.5 | I | | 23 | 55 | 22 |
| 166.10 | I | | 23 | 59 | 18 |

(suite)

**RESISTANCE AU CMV SOUCHE TL28 DE PLANTES DE MELON EN GENERATION T1 EXPRIMANT LE GENE DE LA PROTEINE DE CAPSIDE**

| LIGNEES | CROISEMENT | % CP | % R | % T | % S |
|---|---|---|---|---|---|
| 170.5 | BC | 0.3 | 9 | 0 | 91 |
| 171.7 | I | | 9 | 0 | 91 |
| TEZIER 10 | | | 0 | 0 | 100 |
| VEDRANTAIS | | | 0 | 0 | 100 |
| VIRGOS | | | 9 2 | 0 | 8 |
| FREE CUCUMBER | | | 1 0 0 | 0 | 0 |

**LEGENDE :** I : autofécondation
BC : croisement avec le génotype TEZIER 10 non transformé
% CP : taux d'expression de la protéine de capside par rapport aux protéines totales solubles
% R : pourcentage de plantes résistantes ou sans symptômes
% T : pourcentage de plantes tolérantes
% S : pourcentage de plantes sensibles

* Résistance au CMV souche TL 28 de lignées de melon en génération T1 exprimant le polyribozyme 136 :

Les résultats obtenus pour 13 lignées T1, sont rassemblées dans le tableau 2 et indiquent que :

- après infection avec TL 28, 30 à 87 % des individus T1 sont sans symptôme dans le cas de 10 lignées ;
- le phénomène de "récupération" est peu présent. Seules 4 lignées présentent de 9 à 26 % de plantes T1 tolérantes.

La plupart des lignées exprimant le polyribozyme 136 ont été obtenues par croisement avec la lignée TZ 10 non transformée. La fréquence théorique attendue du polyribozyme 136 est de 50 % dans les plantes T1 inoculées avec le CMV. Le pourcentage de plantes de "type résistant" varie entre 30 et 65 % selon les lignées transgéniques.

En conclusion, pour la stratégie "ribozyme", un plus grand nombre de lignées en génération T1 sont sans symptôme et possèdent très peu de plantes tolérantes.

**TABLEAU 2**

**RESISTANCE AU CMV SOUCHE TL28 DE PLANTES DE MELON EN GENERATION T1 EXPRIMANT LE POLYRIBOZYME 136**

| LIGNEES | CROISEMENT | %R | %T | %S |
|---|---|---|---|---|
| 141.1 | BC | 48 | 9 | 43 |
| 141.2 | BC | 39 | 26 | 35 |
| 141.3 | BC | 43 | 17 | 40 |
| 141.4 | BC | 26 | 26 | 48 |
| 141.5 | BC | 52 | 0 | 48 |
| 141.6 | BC | 39 | 0 | 61 |
| 146.19 | BC | 30 | 0 | 70 |
| 146.28 | BC | 47 | 0 | 53 |
| 146.42 | I | 87 | 0 | 13 |
| 146.47 | BC | 43 | 0 | 57 |
| TEZIER 10 | | 0 | 0 | 100 |
| VEDRANTAIS | | 0 | 0 | 100 |
| VIRGOS | | 92 | 0 | 8 |

(suite)

**RESISTANCE AU CMV SOUCHE TL28 DE PLANTES DE MELON EN GENERATION T1 EXPRIMANT LE POLYRIBOZYME 136**

| LIGNEES | CROISEMENT | %R | %T | %S |
|---|---|---|---|---|
| FREE CUCUMBER | | 100 | 0 | 0 |

**LEGENDE :** I : autofécondation
BC : croisement avec le génotype TEZIER 10 non transformé
% R : pourcentage de plantes résistantes ou sans symptômes
% T : pourcentage de plantes tolérantes
% S : pourcentage de plantes sensibles

* Résumé des tests de résistance au CMV souche TL 28 et de l'évaluation de la ségrégation par test GUS pour certaines lignées :

Les résultats mentionnés dans le tableau 3 montrent :

- l'obtention de deux lignées de type résistantes (plantes sans symptôme et plantes tolérantes) exprimant le gène de la protéine de capside (lignées 88.105 et 153.8), d'une lignée totalement résistante (plantes sans symptôme) exprimant le polyribozyme 136 (lignée 146.42).
- l'obtention de deux lignées tolérantes (plantes tolérantes) exprimant le polyribozyme 136 (lignées 141.1 et 146.28).

## TABLEAU 3

### TEST DE RESISTANCE AU CMV SOUCHE TL28 ET COMPORTEMENTS DU GENE GUS ET DU GENE DE RESISTANCE DANS LA DESCENDANCE

| LIGNEES | X | GEN | CARACTERISTIQUES MOLECULAIRES | %G | R | | | T | | | S | | |
|---|---|---|---|---|---|---|---|---|---|---|---|---|---|
| | | | | | %R | %G | QT. VIRUS | %T | %G | QT. VIRUS | %S | %G | QT. VIRUS |
| 88.105 | | T0 | K+CP+G+ | | | | | | | | | | |
| 702 | I | T1 | K+CP+G+ | 80 | 3 | 100 | 0,78 +/-0,02 | 22 | 100 | 1,46 +/-0,39 | 75 | 53 | 1,42 +/-0,26 |
| 710 | I | T2 | K+CP+G+ | 100 | | | | 100 | 100 | 0,71 +/-0,11 | | | |
| 153.8 | | T0 | K+CP+G+ | | | | | | | | | | |
| 740 | I | T1 | K+CP+G+ | 73 | 12 | 100 | 0,56 +/-0,10 | 43 | 100 | 0,85 +/-0,10 | 45 | 58 | 0,94 +/-0,17 |
| 545 | I | T2 | K+CP+G+ | 100 | 50 | 100 | 0,69 +/-0,56 | 50 | 100 | 0,92 +/-0,57 | | | |
| 159.8 | | T0 | K+CP+G- | | | | | | | | | | |
| 739.1 | I | T1 | K+CP+G- | 0 | | | | | | | 100 | 0 | 1,42 +/-0,27 |

Tableau 3 (suite)

| | | | | | | | | | | | | | |
|---|---|---|---|---|---|---|---|---|---|---|---|---|---|
| 141.1 | | T0 | K+RZ+G+ | | | | | | | | | | |
| 730.1 | BC | T1 | K+RZ+G+ | 57 | | | | 40 | 100 | 0,88 +/-0,21 | 60 | 19 | 1,05 +/-0,17 |
| 146.28 | | T0 | K+RZ+G+ | | | | | | | | | | |
| 734.1 | BC | T1 | K+RZ+G+ | 73 | | | | | | | 100 | 73 | 1,96 +/-0,48 |
| 539.2 | I | T2 | K+RZ+G+ | 68 | | | | 37 | 100 | 1,00 +/-0,25 | 63 | 53 | 1,64 +/-0,42 |
| 146.31 | | T0 | K-RZ-G- | | | | | | | | | | |
| 735.1 | BC | T1 | K-RZ-G- | 0 | | | | | | | 100 | 0 | 1,64 +/-0,46 |
| 146.42 | | T0 | K+RZ+G+ | | | | | | | | | | |
| 755.1 | I | T1 | K+RZ+G+ | 77 | 79 | 100 | 0,42 +/-0,22 | | | | 21 | 0 | 1,75 +/-0,37 |
| 540 | I | T2 | K+RZ+G+ | 82 | 90 | 100 | 0,45 +/-0,30 | | | | 10 | 0 | 1,52 +/-0,65 |
| TEZIER 10 | | | | | 0 | | | 0 | | | 100 | | 1,70 +/-0,77 |
| VEDRANTAIS | | | | | 0 | | | 0 | | | 100 | | 1,70 +/-0,72 |
| TIGOS | | | | | 96 | | 0,05 +/-0,02 | 4 | | | 0 | | |
| FREE CUCUMBER | | | | | 100 | | 0,21 +/-0,10 | 0 | | | 0 | | |

LEGENDE :
: type de croisement. I : autofécondation. BC : croisement avec le génotype TEZIER 10 non transformé
GEN. : génération. T0 : transformant primaire. T1 : descendance n°1. T2 : descendance n°2
K : gène nptII. CP : gène de la protéine de capside. RZ : polyribozyme 136. G : gène gus
+ : présence. - : absence. QT. : quantité
% G : pourcentage de plantes exprimant le gène gus
% R : pourcentage de plantes résistantes ou sans symptômes
% T : pourcentage de plantes tolérantes
% S : pourcentage de plantes sensibles

**1) Etude génétique des lignées :**

Le taux d'expression du gène gus permet de déterminer le type de ségrégation et l'état d'homozygotie.

Pour les deux lignées 88.105 et 153.8, le taux d'expression du gène GUS est de 80 % et 73 % respectivement en génération T1 obtenue par autofécondation. Ceci indique une ségrégation de type mendélien d'un gène dominant (3 : 1) avec l'intégration du T-DNA en un seul locus. Des analyses moléculaires ont montré la présence d'une seule copie intègre du T-DNA dans le génome végétal.

Par ailleurs, le taux d'expression du gène gus est de 100 % en génération T2 pour les deux lignées, ce qui souligne l'état d'homozygotie du gène intégré.

Pour la lignée 146.42, le taux d'expression du gène gus est de 77 % en génération T1 obtenue par autofécondation ce qui montre une ségrégation de type mendélien d'un gène dominant avec intégration du T-DNA en un seul locus. Des analyses moléculaires ont montré la présence de plusieurs T- DNA et/ou fragments de T-DNA en un seul locus du génome végétal.

Par ailleurs, le taux d'expression du gène gus étant de 90 % en génération T2 souligne que la lignée testée n'est pas homozygote pour ce gène intégré (tableau 3). L'homozygotie a été obtenue en T3 pour la lignée 146.42. Pour la lignée 141.1 le taux d'expression du gène gus est de 57 % en génération TEZIER 10 non transformé. Ceci correspond encore à une ségrégation de type mendélien d'un gène dominant (1 : 1) avec une intégration du T-DNA en un seul locus. Des analyses moléculaires ont montré la présence d'un seul T-DNA intègre dans le génome végétal.

Pour la lignée 146.28, le taux d'expression du gène gus est de 73 % en génération T1 obtenue après croisement avec le génotype TEZIER 10 non transformé. Ceci indique une ségrégation des gènes dominants avec intégration du T-DNA en plusieurs loci. Des analyses moléculaires ont montré la présence de plusieurs T-DNA et ou fragments de T-DNA en deux loci de génome végétal.

**2) Comportement vis à vis du virus :**

Toutes les plantes de type résistant expriment le gène GUS. Parmi les plantes sensibles certaines expriment le gène GUS (tableau 3).

Pour la lignée 88.105, 25 % de plantes de type résistant et 75 % de plantes sensibles ont été obtenues en génération T1 résultant d'une autofécondation. Ceci montre une ségrégation de type mendélien du gène récessif (1 : 3) pour le gène de résistance.

Pour la lignée 153.8, 55 % de plantes de type résistant et 45 % de plantes sensibles ont été obtenues en génération T1 résultant d'une autofécondation. Il est difficile de conclure quant au comportement du gène de résistance.

Pour la lignée 141.1, 40 % de plantes tolérantes et 60 % de plantes sensibles ont été obtenues en génération T1 par croisement avec le génotype TEZIER 10 non transformé. Ceci indique une ségrégation de type mendélien d'un gène dominant (1 : 1) pour le gène de résistance. Le comportement du polyribozyme 136 est donc similaire à celui du gène gus.

Pour la lignée 146.42, 79 % de plantes sans symptôme et 21 % des plantes sensibles ont été obtenues en génération T1 résultant d'une autofécondation. Ceci implique une ségrégation de type mendélien d'un gène dominant (3 : 1) pour le gène de résistance. Le comportement du ribozyme 136 est donc similaire à celui du gène gus.

**3) Evaluation des quantités de virus :**

Les quantités de virus détectées par test ELISA sont peu élevées dans les plantes sans symptôme mais néanmoins supérieures à celles du témoin résistant Free cucumber (Tableau 10).

Les plantes tolérantes renferment des quantités de virus non négligeables.

Les plantes sensibles possèdent des quantités de virus très élevées.

Les plantes exprimant les polyribozymes (lignées 146.42, T2 et T3) contiennent une quantité moins importante de virus que les plantes de types résistants exprimant la protéine de capside (lignée 153.8 T2).

Il apparaît que les quantités de virus détectées sont relativement bien corrélées à la sévérité des symptômes.

**4) Développement des symptômes du CMV au cours du temps :**

Des plantes T1 des lignées mentionnées dans les figures 1 et 2 ont été infectées avec du CMV souche TL 28. Les symptômes ont été comptabilisés 6, 8, 12, 14, 16 et 19 jours après inoculation. Les résultats sont présentés dans les histogrammes (figures 9 et 10).

Les valeurs obtenues après J16 ne varient plus. Dans le cas des lignées "protéine de capside", il est à noter une diminution plus ou moins rapide du nombre de plantes sans symptôme, au profit de l'apparition de plantes avec symptômes. Pour les lignées 88-105 et 153-8 en génération T1, à J16 et J14 respectivement il se développe des plantes tolérantes caractérisées par le phénomène de "récupération". De plus, chez la lignée 88-105 en génération T2, 100 % des plantes sans symptôme à J8 se convertissent en 100 % de plantes tolérantes qui subsistent jusqu'à J19 (figure 9).

Ce changement brutal des plantes sans symptôme en plantes tolérantes peut s'expliquer par un fort effet des conditions climatiques.

Chez la lignée 153.8 en génération T2, il est à noter une apparition progressive de plantes tolérantes. A partir de J16, 50 % des plantes T2 sont sans symptôme et 50 % sont tolérantes.

Dans le cas des lignées "polyribozyme", il est à souligner une diminution du nombre de plantes T1 sans symptôme au profit des plantes avec symptômes chez les lignées 141.1 et 146.28 (figure 10).

De plus, pour 141.1 il est à noter la présence de 40 % de plantes T1 tolérantes à partir de J16. Dans la lignée 146.28, des plantes T2 tolérantes se développent à partir de J14 pour atteindre 26 % en J16.

Par ailleurs, dans la lignée 146.42, les plantes T1 sans symptôme représentent 80 % en J6 et restent presque stables au cours du temps (78 % en J8, J12 et J 14, 79 % dès J16).

Les plantes T2 de la lignée 146.42 ont un comportement similaire. Ce résultat montre un très bon niveau de résistance de cette lignée et une stabilité du gène de résistance dans la descendance.

En conclusion, deux lignées de "type résistantes" exprimant le gène de la protéine de capside et une lignée totalement résistante, exprimant le polyribozyme 136 ont été obtenues. Pour les deux lignées exprimant le gène de la protéine de capside la résistance observée s'apparente plus à une tolérance qu'à une résistance totale. En effet, certaines plantes infectées par le virus qui présentent de forts symptômes, peuvent dans certaines conditions développer de nouvelles feuilles saines.

Pour la lignée totalement résistante exprimant le polyribozyme 136 aucun phénomène de tolérance n'est observé. Pour ces 2 lignées exprimant la protéine de capside, des quantités de virus relativement élevées ont été observées dans les plantes résistantes, alors que dans les plantes résistantes exprimant le polyribozyme 136, la quantité de virus est presque égale au témoin résistant Free Cucumber.

## EXEMPLE 5 : RESISTANCE A L'INFECTION PAR LE CMV SOUCHE TL 28 DE LIGNEES DE MELON EN GENERATION T1 EXPRIMANT LE POLYRIBOZYME 165 :

Les résultats obtenus pour 13 lignées T1, exprimant le polyribozyme 165, sont rassemblés dans le tableau 4 et indiquent que :

- après infection avec TL28, 15 à 100% des individus T1 sont sans symptôme dans le cas de 12 lignées ;
- le phénomène de "récupération" est peu présent. Seules 5 lignées présentent de 6 à 60% de plantes T1 tolérantes.

La plupart des lignées exprimant le polyribozyme 165 ont été obtenues par autofécondation. La fréquence théorique attendue du polyribozyme 165 est de 75% dans les plantes T1 inoculées avec le CMV. Le pourcentage de plantes de "type résistant" varie entre 15 et 100% selon les lignées transgéniques.

En conclusion, comme dans le cas des lignées exprimant le polyribozyme 136, un grand nombre de lignées exprimant le polyribozyme 165 sont sans symptôme et possèdent peu de plantes tolérantes. Le polyribozyme 165 diffère du polyribozyme 136 par 2 ribozymes fonctionnels supplémentaires. Les 2 constructions conduisent à l'obtention de résultats similaires pour la résistance à l'infection par le CMV.

**TABLEAU 4**

**RESISTANCE AU CMV SOUCHE TL28 DE PLANTES DE MELON EN GENERATION T1 EXPRIMANT LE POLYRIBOZYME 165**

| LIGNEES | CROISEMENT | %R | %T | %S |
|---|---|---|---|---|
| 10.2 | I | 100 | 0 | 0 |
| 203.6 | BC | 0 | 0 | 100 |
| 205.1 | I | 50 | 45 | 5 |
| 205.3 | I | 66 | 0 | 34 |
| 206.1 | I | 94 | 6 | 0 |
| 207.3 | I | 61 | 0 | 39 |
| 207.5 | I | 80 | 0 | 20 |
| 207.7 | I | 64 | 0 | 36 |
| 207.8 | I | 69 | 9 | 22 |
| 207.9 | I | 100 | 0 | 0 |
| 211.1 | I | 15 | 0 | 85 |
| 212.1 | I | 87 | 13 | 0 |
| 215.1 | I | 20 | 60 | 20 |
| VEDRANTAIS | | 0 | 0 | 100 |
| VIRGOS | | 100 | 0 | 0 |

(suite)

**RESISTANCE AU CMV SOUCHE TL28 DE PLANTES DE MELON EN GENERATION T1 EXPRIMANT LE POLYRIBOZYME 165**

| LIGNEES | CROISEMENT | %R | %T | %S |
|---|---|---|---|---|
| FREE CUCUMBER | | 100 | 0 | 0 |

LEGENDE : I : autofécondation
BC : croisement avec le génotype TEZIER 10 non transformé
% R : pourcentage de plantes résistantes ou sans symptômes
% T : pourcentage de plantes tolérantes
% S : pourcentage de plantes sensibles

## Revendications

1. Polyribozyme ayant une activité d'endoribonucléase et étant capable d'inactiver le gène de la protéine de capside d'un virus, **caractérisé en ce que** ledit polyribozyme comprend une pluralité de ribozymes, chacun clivant ledit gène de protéine de capside, ou le transcrit correspondant, ou son intermédiaire de réplication, chaque ribozyme comprenant une région catalytique et des bras hybridants, où lesdits bras hybridants sont complémentaires d'un fragment du gène de la protéine de capside.

2. Polyribozyme selon la revendication 1 de structure :

$$[(Y_1 - Q - Y_2) - (S)_n]_p$$

dans laquelle $Y_1$ et $Y_2$ sont chacun les bras hybridants ayant au moins 4 bases et étant complémentaires à des fragments distincts, consécutifs et contigus du gène de la protéine de capside, du transcrit correspondant ou de l'intermédiaire de réplication correspondant au gène de la protéine de capside ;
Q est la région catalytique d'un ribozyme,
S est une séquence qui a une longueur entre 2 et 500 bases et qui est non complémentaire au gène de la protéine de capside, au transcrit correspondant, et à son intermédiaire de réplication ;
n= 0 ou 1 ;
p est un entier supérieur à 1.

3. Polyribozyme selon la revendication 1 ou 2, **caractérisé en ce que** lesdites régions catalytiques proviennent de ribozymes du type « Tête de marteau » (hammerhead), ou du type « Epingle à cheveux » (hairpin).

4. Polyribozyme selon la revendication 3, où chaque ribozyme cible un site XUX dans le gène ou dans le transcrit correspondant.

5. Polyribozyme selon la revendication 4, où le nombre de ribozymes est égal ou inférieur au nombre total de sites XUX présents dans le gène de la protéine de capside, ou dans le transcrit correspondant du gène de la protéine de capside, ou dans l'intermédiaire de réplication.

6. Polyribozyme selon la revendication 5, où chaque ribozyme cible un site XUX dans le gène ou dans le transcrit correspondant, qui fait partie des zones d'homologie conservées entres différentes souches d'un même virus, ou entre différents virus apparentés.

7. Polyribozyme selon l'une quelconque des revendications précédentes, où les bras hybridants sont complémentaires du gène de la protéine de capside d'un virus végétal choisi parmi les groupes de virus suivants : les Caulimovirus, les Geminivirus, les Reoviridae, les Rhabdoviridae, les Tobamovirus, les Potexvirus, les Potyvirus, les Carlavirus, les Closterovirus, les Tobravirus, les Hordeivirus, les Tymovirus, les Lutéovirus, les Tombusvirus, les Sobemovirus, les Népovirus, les Comovirus, les Cucumovirus, les Bromovirus et les Ilarvirus.

8. Polyribozyme selon la revendication 7, où ledit virus végétal est choisi parmi le groupe suivant : le virus de la mosaïque du chou-fleur (CaMV), le virus du Streak du Mais (MSV), le virus des tumeurs de blessures (WTV), le Potato Yellow Dwarf Virus (PYDV), le Tomato Spotted Witt Virus (TSWV), le virus de la maladie bronzée de la tomate, le virus de la mosaïque du tabac (TMV), le virus X de la pomme de terre (PXV), le virus Y de la pomme de terre (PYV), le virus latent de l'oeillet (CLV), le virus de la jaunisse de la betterave (BYV), le virus du Rattle du tabac (TRV), le virus de la Mosaïque striée de l'orge, le virus de la mosaïque jaune du navet (TYMV), le virus de la jaunisse nanisante de l'orge (BYDV), le virus de l'enroulement de la pomme de terre (PLRV), le Tomato Bushy Stunt Virus (TBSV), le Southern Bean Mosaïc Virus (SBMV), le virus de la nécrose du tabac (TNV), le virus des taches en anneaux du tabac (TRSV), le virus de la mosaïque du Vigna (CPMV), le virus de la mosaïque enation du pois (PEMV), le virus de la mosaïque du concombre (CMV), le virus de la mosaïque du Brome (BMV) et le virus du Streak du tabac (TSV).

**9.** Polyribozyme selon la revendication 8, où les bras hybridants sont complémentaires du transcrit du gène de la protéine de capside du CMV.

**10.** Polyribozyme selon l'une quelconque des revendications précédentes, constitué d'ARN.

**11.** Séquence d'ADN codant pour un polyribozyme selon l'une quelconque des revendications 1 à 10.

**12.** Procédé pour rendre une plante résistante à un virus, comprenant l'introduction dans la plante d'un polyribozyme selon l'une quelconque des revendications 1 à 10, ou d'une séquence codant pour un polyribozyme selon l'une quelconque des revendications 1 à 10.

**13.** Procédé selon la revendication 12, où l'introduction du polyribozyme s'effectue par la transformation génétique, d'une partie de la plante, par une séquence d'ADN codant pour le polyribozyme, suivie par la régénération d'une plante transgénique.

**14.** Procédé selon la revendication 13, où la transformation s'effectue par l'intermédaire d'Agrobacterium tumefaciens ou Agrobacterium rhizogenes.

**15.** Plante transgénique résistante à un virus, **caractérisée en ce qu'**elle contient dans son génome une séquence qui donne lieu, par transcription, à un polyribozyme selon, l'une quelconque des revendications 1 à 10.

**16.** Plante transgénique selon la revendication 15, ladite plante étant résistante au CMV.

**17.** Plante transgénique selon la revendication 16, **caractérisée en ce qu'**il s'agit du melon, du concombre, de la courgette, de la tomate, du poivron ou du haricot.

**18.** Fruits de plantes transgéniques selon l'une quelconque des revendications 15 à 17.

**19.** Semences de plantes transgéniques selon l'une quelconque des revendications 15 à 17.

**20.** Cellules végétales transformées par la séquence de la revendication 11.

## Claims

**1.** Polyribozyme having endoribonuclease activity and being capable of inactivating the capsid protein gene of a virus, **characterised in that** said polyribozyme comprises a plurality of ribozymes, each of which cleaves the said capsid protein gene or the corresponding transcript, or its replication intermediate,
each ribozyme comprising a catalytic region and hybridizing arms, wherein the hybridizing arms are complementary to a fragment of the capsid protein gene.

**2.** Polyribozyme according to claim 1 having the formula :

$$[(Y_1 - Q - Y_2) - (S)_n]_p$$

wherein $Y_1$ and $Y_2$ are each hybridizing arms having at least 4 bases and being complementary to distinct fragments, consecutive and adjoining, of the capsid protein gene, of the corresponding transcript or of the replication intermediate corresponding to the capsid protein gene ;

Q is the catalytic region of a ribozyme,
S is a nucleotide sequence which has a length comprised between 2 and 500 bases and is not complementary to the capsid protein gene, the corresponding transcript or the replication intermediate
n = 0 or 1 ;
p is an integer greater than 1.

**3.** Polyribozyme according to claim 1 or 2 **characterised in that** said catalytic regions are selected from the group consisting of a hammerhead-type ribozyme and a hairpin type ribozyme.

**4.** Polyribozyme according to claim 3 wherein each ribozyme targets an XUX site in the gene or the corresponding transcript.

**5.** Polyribozyme according to claim 4 wherein the number of ribozymes is equal to or less than the total number of XUX sites present in the capsid protein gene, or in the corresponding transcript of the capsid protein gene, or in the replication intermediate.

**6.** Polyribozyme according to claim 5, wherein each ribozyme targets an XUX site in the gene or in the corresponding transcript, which occurs in a zone of homology conserved between different strains of the same virus or between different related viruses.

**7.** Polyribozyme according to any one of the preceding claims wherein the hybridizing arms are complementary to the capsid protein gene of a plant virus chosen from the group consisting of :

the Caulimoviruses, the Geminiviruses, the Reoviridae, the Rhabdoviridae, the Tobamoviruses, the Potexviruses, the Potyviruses, the Carlaviruses, the Closteroviruses, the Tobraviruses, the Hordeiviruses, the Tymoviruses, the Luteoviruses, the Tombusviruses, the Sobemoviruses, the Nepoviruses, the Comoviruses, the Cucumoviruses, the Bromoviruses and the Ilarviruses.

**8.** Polyribozyme according to claim 7, wherein the plant virus is chosen from the group consisting of :

Cauliflower Mosaic Virus (CaMV), Maize Streak Virus (MSV), Wound Tumour Virus (WTV), Potato Yellow Dwarf Virus (PYDV), Tomato Spotted Wilt Virus (TSWV), Tobacco Mosaic Virus (TMV), Potato Virus X (PXV), Potato Virus Y (PYV), Carnation Latent Virus (CLV), Beet Yellow Virus (BYV), Tobacco Rattle Virus (TRV), Barley Stripe Mosaic Virus (BSMV), Turnip Yellow Mosaic Virus (TYMV), Barley Yellow Dwarf Virus (BYDV), Potato Leaf Roll Virus (PLRV), Tomato Bushy Stunt Virus (TBSV), Southern Bean Mosaic Virus (SBMV), Tobacco Necrosis Virus (TNV), Tobacco Ring Spot Virus (TRSV), Cow Pea Mosaic Virus (CPMV), Pea Enation Mosaic Virus (PEMV), Cucumber Mosaic Virus (CMV), Brome Mosaic virus (BMV) and Tobacco Streak Virus (TSV).

**9.** Polyribozyme according to claim 8 wherein the hybridizing arms are complementary to the transcript of the capsid protein gene of the CMV.

**10.** Polyribozyme according to any one of the preceding claims, consisting of RNA.

**11.** DNA molecule coding for a polyribozyme according to any one of claims 1 to 10.

**12.** Process for rendering a plant resistant to a virus, which comprises the introduction into the plant of a polyribozyme according to any one of claims 1 to 10 or a nucleic acid molecule encoding a polyribozyme according to any one of claims 1 to 10

**13.** Process according to claim 12 wherein the introduction into the plant of the polyribozyme is made by genetic transformation of a part of the plant by a DNA molecule coding for the polyribozyme, followed by regeneration of a transgenic plant.

**14.** Process according to claim 13, wherein the transformation is carried out by the intermediary of Agrobacterium tumefaciens or Agrobacterium rhizogenes.

**15.** Transgenic plant resistant to a virus, **characterised in that** said plant contains in its genome a sequence which gives rise on transcription to a polyribozyme according to any one of claims 1 to 10.

**16.** Transgenic plant according to claim 15, said plant being resistant to CMV.

**17.** Transgenic plant according to claim 16 which is a melon, a cucumber, a courgette, a tomato, a sweet pepper or a bean.

**18.** Fruits of transgenic plants according to any one of claims 15 to 17.

**19.** Seed of transgenic plants according to any one of claims 15 to 17.

**20.** Plant cells transformed by the DNA molecule according to claim 11.

**Patentansprüche**

**1.** Polyribozym, das eine Endoribonucleaseaktivität aufweist und in der Lage ist, das Gen des Capsidproteins eines Virus zu inaktivieren, **dadurch gekennzeichnet, dass** dieses Polyribozym eine Vielzahl von Ribozymen umfasst, von denen jedes das Gen des Capsidproteins oder das entsprechende Transkript oder seinen Replikationsvermittler spaltet, wobei jedes Ribozym eine katalytische Region und hybridisierende Arme umfasst, wobei diese hybridisierenden Arme komplementär zu einem Fragment des Gens des Capsidproteins sind.

**2.** Polyribozym gemäß Anspruch 1 der Struktur:

$$[(Y_1 - Q - Y_2) - (S)_n]_p$$

worin $Y_1$ und $Y_2$ jeweils die hybridisierenden Arme sind, welche mindestens 4 Basen aufweisen und komplementär sind zu bestimmten, aufeinander folgenden und aneinander grenzenden Fragmenten des Gens des Capsidproteins, des entsprechenden Transkripts oder des Replikationsvermittlers, der dem Gen des Capsidproteins entspricht;
Q die katalytische Region eines Ribozyms ist,
S eine Sequenz ist, die eine Länge zwischen 2 und 500 Basen hat und die nicht komplementär zum Gen des Capsidproteins, zum entsprechenden Transkript oder seinem Replikationsvermittler ist;
n = 0 oder 1;
p eine ganze Zahl größer 1 ist.

**3.** Polyribozym gemäß Anspruch 1 oder 2, **dadurch gekennzeichnet, dass** die katalytischen Regionen von Ribozymen des Typs "Hammerkopf" (hammerhead) oder vom Typ "Haarnadel" (hairpin) stammen.

**4.** Polyribozym gemäß Anspruch 3, worin jedes Ribozym auf eine Stelle XUX in dem Gen oder in dem entsprechenden Transkript zielt.

**5.** Polyribozym gemäß Anspruch 4, worin die Zahl der Ribozyme gleich oder geringer ist als die Gesamtzahl der Stellen XUX, die in dem Gen des Capsidproteins oder in dem entsprechenden Transkript des Gens des Capsidproteins oder im Replikationsvermittler vorliegen.

**6.** Polyribozym gemäß Anspruch 5, worin jedes Ribozym auf eine Stelle XUX in dem Gen oder in dem entsprechenden Transkript zielt, die zu homologen Bereichen gehören, die zwischen verschiedenen Stämmen desselben Virus oder zwischen verschiedenen verwandten Viren konserviert sind.

**7.** Polyribozym gemäß einem der vorigen Ansprüche, worin die hybridisierenden Arme komplementär zum Gen des Capsidproteins eines pflanzlichen Virus sind, der unter folgenden Virusgruppen ausgewählt ist: Caulimoviren, Geminiviren, Reoviridae, Rhabdoviridae, Tobamoviren, Potexviren, Potyviren, Carlaviren, Closteroviren, Tobraviren, Hordeiviren, Tymoviren, Luteoviren, Tombusviren, Sobemoviren, Nepoviren, Comoviren, Cucumoviren, Bromoviren und Ilarviren.

**8.** Polyribozym gemäß Anspruch 7, wobei der Pflanzenvirus ausgewählt ist aus folgender Gruppe: Blumenkohlmosaikvirus (CaMV), Virus der Strichelkrankheit des Mais (MSV), Wundtumorvirus (WTV), Potato Yellow Dwarf Virus (PYDV), Tomato Spotted Wilt Virus (TSWV), der Virus der Bronzefleckenkrankheit der Tomate, Tabakmosaikvirus (TMV), Kartoffel-X-Virus (PXV), Kartoffel-Y-Virus (PYV), Latentvirus der Nelke (CLV), Rübenvergilbungsvirus (BYV), Tabak-Rattle-Virus (TRV), Streifen-Mosaikvirus der Gerste, Gelbmosaikvirus der Weißrübe (TYMV), Gelbverzwergungsvirus der Gerste (BYDV), Kartoffelblattrollvirus (PLRV), Tomato Bushy Stunt Virus (TBSV), Southern Bean-Mosaikvirus (SBMV), Tabaknekrosevirus (TNV), Tabak-Ringfleckenvirus (TRSV), Mosaikvirus der Augenbohne (CPMV), Erbsen-Enationenmosaikvirus (PEMV), Gurkenmosaikvirus (CMV), Trespenmosaikvirus (BMV) und Tobacco Streak Virus (TSV).

**9.** Polyribozym gemäß Anspruch 8, wobei die hybridisierenden Arme komplementär zum Transkript des Gens des Capsidproteins von CMV sind.

**10.** Polyrlbozym gemäß einem der vorigen Ansprüche, bestehend aus RNA.

**11.** DNA-Sequenz, die für ein Polyribozym gemäß einem der Ansprüche 1 bis 10 codiert.

**12.** Verfahren, eine Pflanze gegen ein Virus resistent zu machen, umfassend das Einbringen eines Polyribozyms gemäß einem der Ansprüche 1 bis 10 oder einer Sequenz, die für ein Polyribozym gemäß einem der Ansprüche 1 bis 10 codiert, in die Pflanze.

**13.** Verfahren gemäß Anspruch 12, wobei das Einbringen des Polyribozyms durch genetische Transformation eines Teils der Pflanze mit einer DNA-Sequenz, die für das Polyribozym codiert, erfolgt, gefolgt von der Regeneration einer transgenen Pflanze.

**14.** Verfahren gemäß Anspruch 13, wobei die Transformation durch die Vermittlung von *Agrobacterium tumefaciensoder Agrobacterium rhizogenes* erfolgt.

**15.** Transgene Pflanze, die gegen einen Virus resistent ist, **dadurch gekennzeichnet, dass** sie in ihrem Genom eine Sequenz enthält, die mittels Transkription ein Polyribozym gemäß einem der Ansprüche 1 bis 10 entstehen lässt.

**16.** Transgene Pflanze gemäß Anspruch 15, wobei diese Pflanze resistent gegen CMV ist.

**17.** Transgene Pflanze gemäß Anspruch 16, **dadurch gekennzeichnet, dass** es sich um Melone, Gurke, Zucchini, Tomate, Paprikaschote oder Bohne handelt.

**18.** Früchte von transgenen Pflanzen gemäß einem der Ansprüche 15 bis 17.

**19.** Saatgut von transgenen Pflanzen gemäß einem der Ansprüche 15 bis 17.

**20.** Pflanzliche Zellen, die mit der Sequenz des Anspruchs 11 transformiert sind.

FIGURE 1A

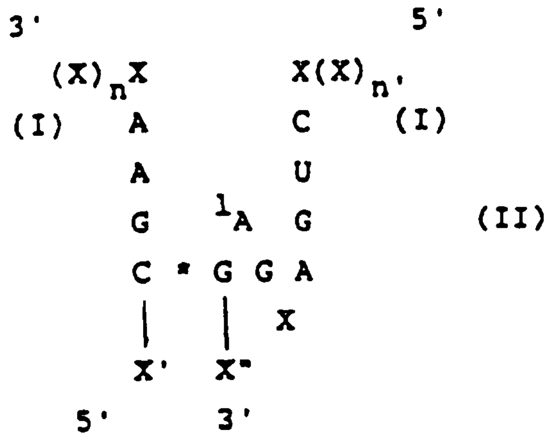


FIGURE 1B

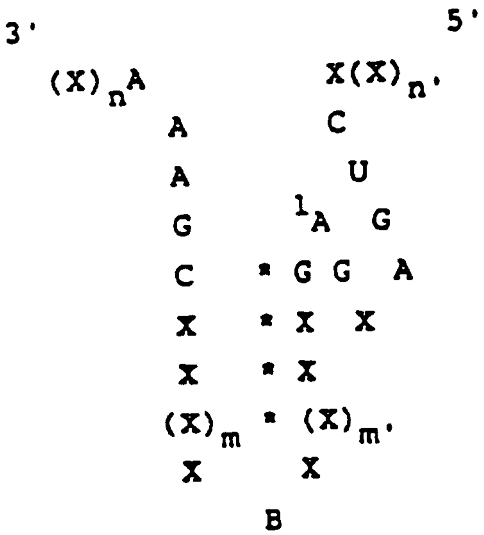

FIGURE 1C

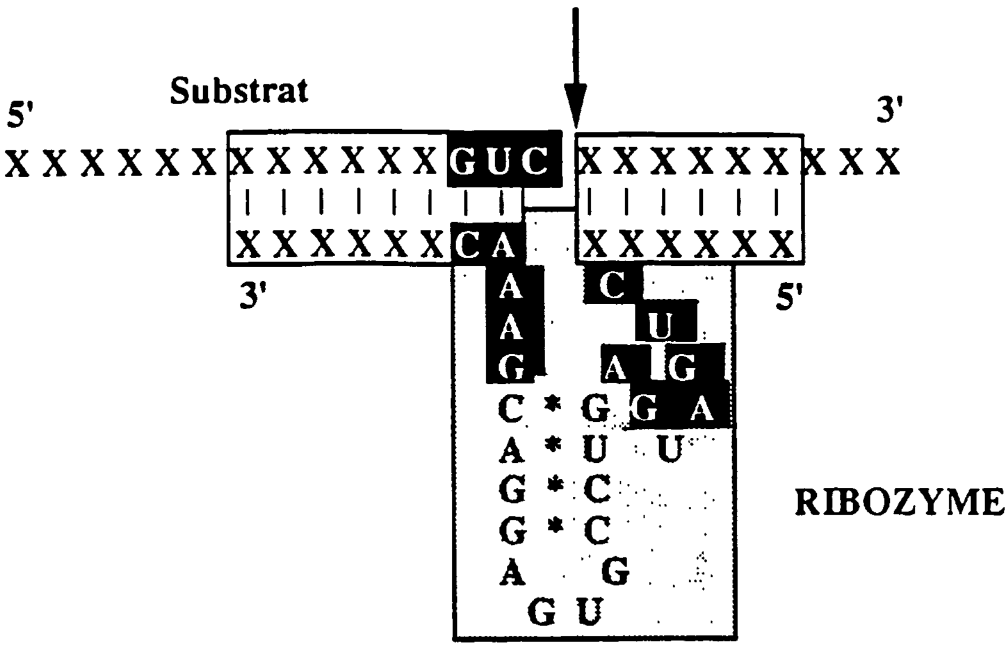


FIGURE 1D

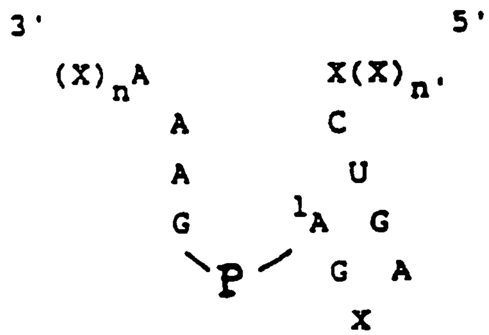

```
AGAGAGTGTGTGTGCTGTGTTTTCTCTTTTGTGTCGTAGAATTGAGTCGAG        51
5'      Oligo n°13                 3'
                                                84
TC ATG GAC AAA TCT GAA TCA ACC AGT GCT GGT CGT AAC         89
   Met Asp Lys Ser Glu Ser Thr Ser Ala Gly Arg Asn         12
                          108
CGT CGA CGT CGT CCG CGT CGT GGT TCC CGC TCC GCC CCC       128
Arg Arg Arg Arg Pro Arg Arg Gly Ser Arg Ser Ala Pro        25

TCC TCC GCG GAT GCT AAC TTT AGA GTC TTG TCG CAG CAG       167
Ser Ser Ala Asp Ala Asn Phe Arg Val Leu Ser Gln Gln        38
                                                204
CTT TCG CGA CTT AAT AAG ACG TTA GCA GCT GGT CGT CCA       206
Leu Ser Arg Leu Asn Lys Thr Leu Ala Ala Gly Arg Pro        51

ACT ATT AAC CAC CCA ACC TTT GTA GGG AGT GAA CGC TGT       245
Thr Ile Asn His Pro Thr Phe Val Gly Ser Glu Arg Cys        64

AGA CCT GGG TAC ACG TTC ACA TCT ATT ACC CTA AAG CCA       284
Arg Pro Gly Tyr Thr Phe Thr Ser Ile Thr Leu Lys Pro        77

CCA AAA ATA GAC CGT GGG TCT TAT TAC GGT AAA AGG TTG       323
Pro Lys Ile Asp Arg Gly Ser Tyr Tyr Gly Lys Arg Leu        90
                                              357  Hind III
TTA CTA CCT GAT TCA GTC ACG GAA TAT GAT AAG AAG CTT       362
Leu Leu Pro Asp Ser Val Thr Glu Tyr Asp Lys Lys Leu       103

GTT TCG CGC ATT CAA ATT CGA GTT AAT CCT TTG CCG AAA       401
Val Ser Arg Ile Gln Ile Arg Val Asn Pro Leu Pro Lys       116
```

```
TTT GAT TCT ACC GTG TGG GTG ACA GTC CGT AAA GTT CCT        440
Phe Asp Ser Thr Val Trp Val Thr Val Arg Lys Val Pro        129

GCC TCC TCG GAC TTA TCC GTT GCC GCC ATC TCT GCT ATG        479
Ala Ser Ser Asp Leu Ser Val Ala Ala Ile Ser Ala Met        142

TTC GCG GAC GGA GCC TCA CCG GTA CTG GTT TAT CAG TAT        518
Phe Ala Asp Gly Ala Ser Pro Val Leu Val Tyr Gln Tyr        155

GCC GCA TCT GGA GTC CAA GCC AAC AAC AAA CTG TTG TAT        557
Ala Ala Ser Gly Val Gln Ala Asn Asn Lys Leu Leu Tyr        168

GAT CTT TCG GCG ATG CGC GCT GAT ATA GGT GAC ATG AGA        596
Asp Leu Ser Ala Met Arg Ala Asp Ile Gly Asp Met Arg        181
             608
AAG TAC GCC [GTC] CTC GTG TAT TCA AAA GAC GAT GCG CTA      635
Lys Tyr Ala Val Leu Val Tyr Ser Lys Asp Asp Ala Leu        194

GAG ACG GAC GAG CTA GTA CTT CAT GTT GAC ATC GAG CAC        674
Glu Thr Asp Glu Leu Val Leu His Val Asp Ile Glu His        207
        Oligo n°14
3' <-------------------------- 5'
CAA CGC ATT CCC ACG TCT GGA GTG CTC CCA GTC TGATTCGT       715
Gln Arg Ile Pro Thr Ser Gly Val Leu Pro Val                218
```

FIGURE 2 (b)

| | |
|---|---|
| GTTCCCAGAATCCTCCCTCCGATCTCTGTGGCGGGAGCTGAGTTGGCAGTTC | 767 |
| TGCTATAAACTGTCTGAAGTCACTAAACGTTTTTACGGTGAACGGGTTGTCC | 819 |
| ATCCAGCTTACGGCTAAAATGGTCAGTCGTGGAGAAATCCACGCCAGTAGAT | 871 |
| TTACAAATCTCTGAGGCGCCTTTGAAACCATCTCCTAGGTTTCTTCGGAAGG | 923 |
| ACTTCGGTCCGTGTACCTCTAGCACAACGTGCTAGTTTCAGGGTACGGGTGC | 975 |
| CCCCCCACTTTCGTGGGGGCCTCCAAAAGGAG | 1007 |

FIGURE 2C

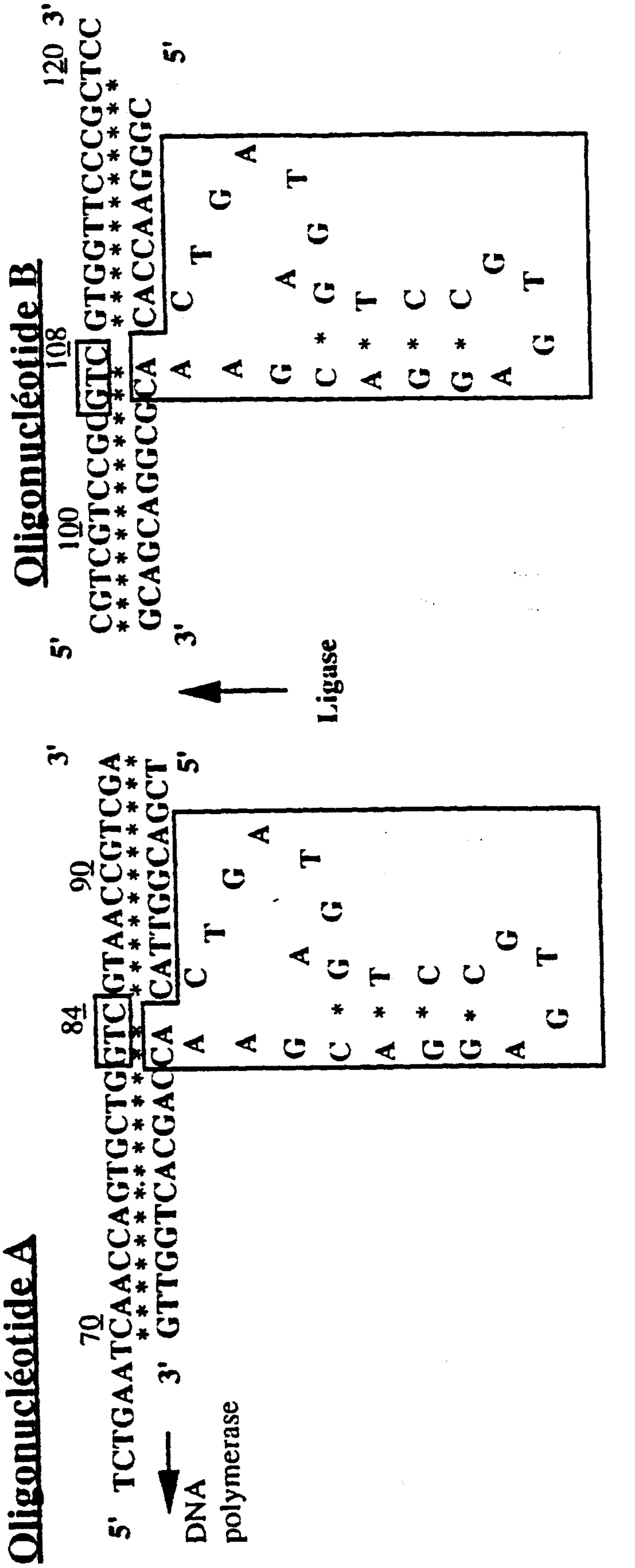
Oligonucléotide A
Oligonucléotide B
5' TCTGAATCAACCAGTGCTGGTCGTAACCGTCGA 3'
3' GTTGGTCACGACCACATTGGCAGCT 5'
70
84
90
5' CGTCGTCCGCGTCGTGGTTCCCGCTCC 3'
3' GCAGCAGGCGCACACCAAGGGC 5'
100
108
120
DNA polymerase
Ligase

FIGURE 3 (a)

## Oligonucléotide C

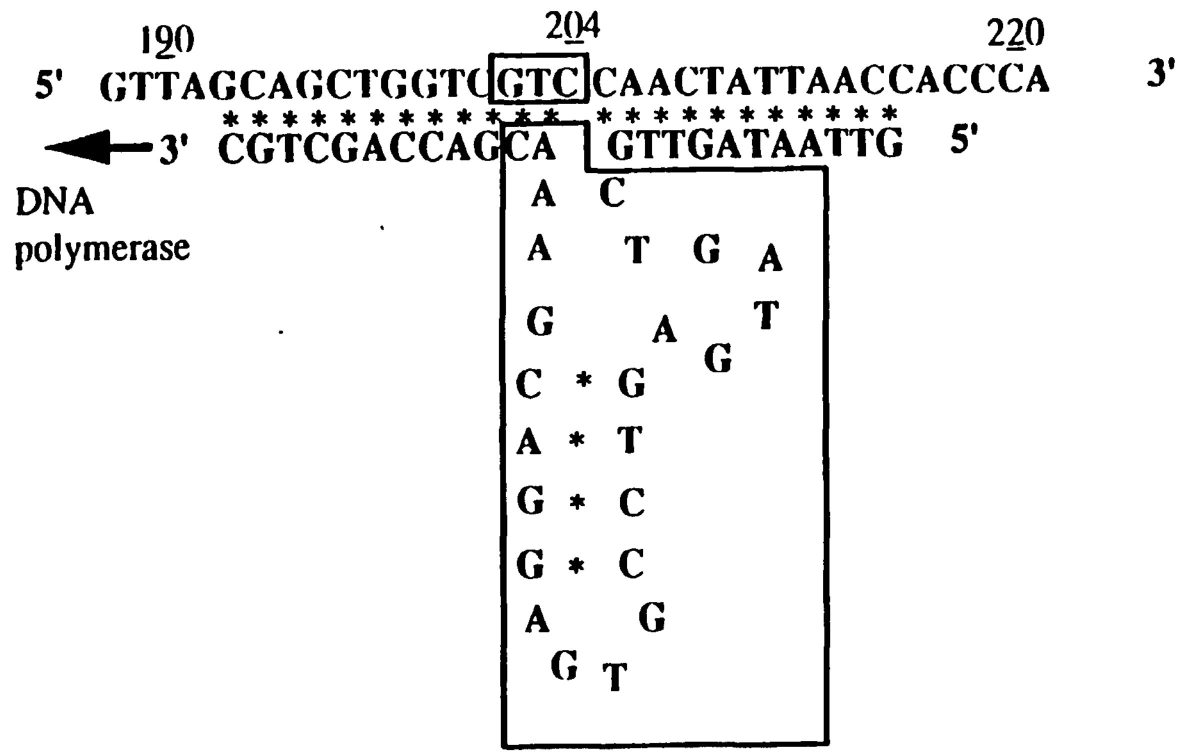


FIGURE 3 (b)

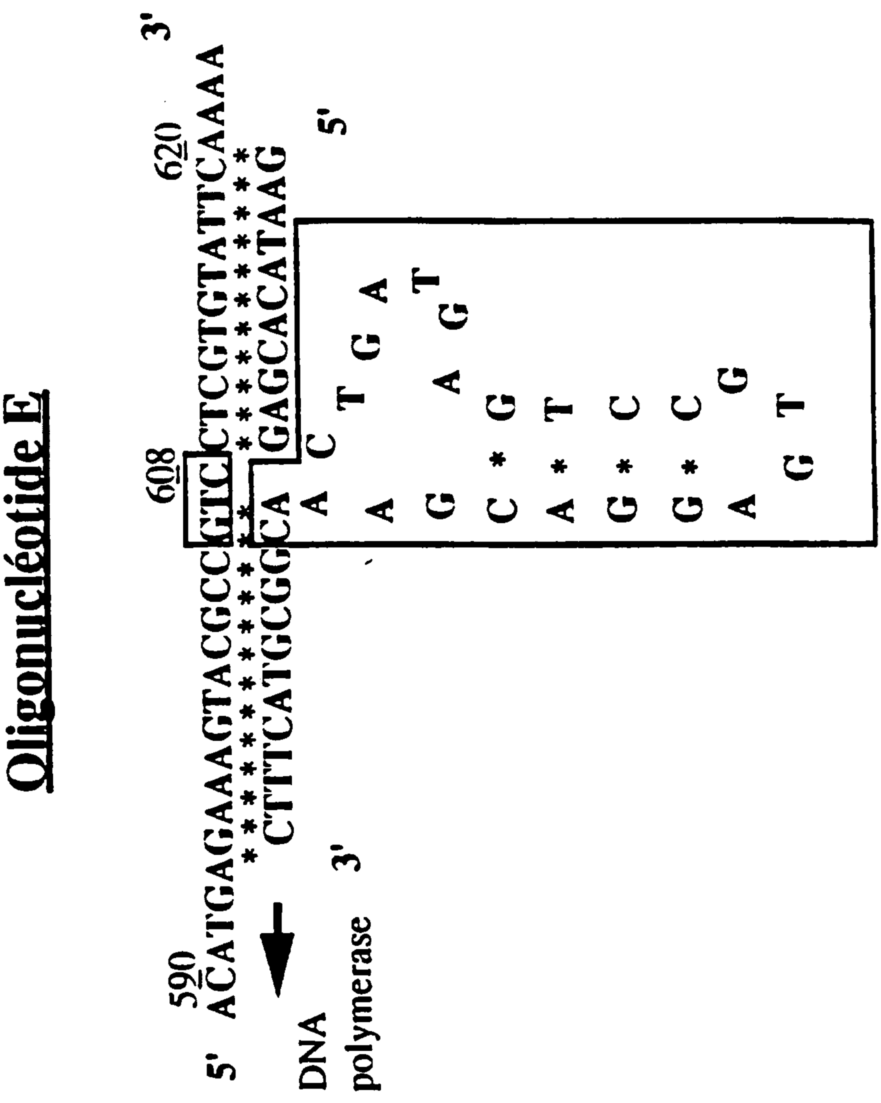
Oligonucléotide E
590
608
620
5' ACATGAGAAAGTACGCCGTCCTCGTGTATTCAAAA 3'
3' CTTTCATGCGGCA
GAGCACACATAAG 5'
DNA polymerase

FIGURE 3 ([illegible])

FIGURE 4 (a)

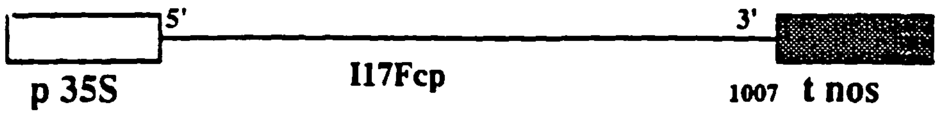


FIGURE 4 (b)

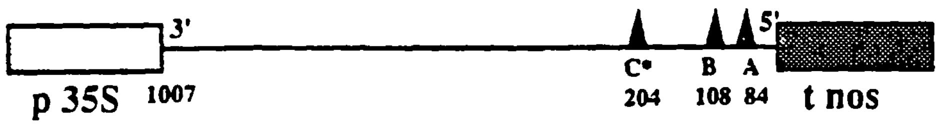


FIGURE 4 (c)

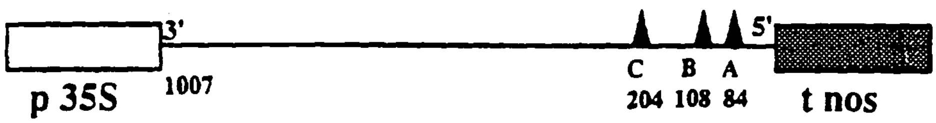


FIGURE 4 (d)

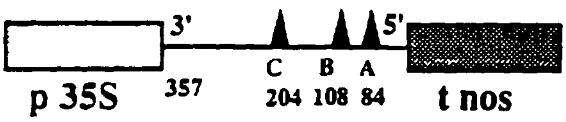


FIGURE 4 (e)

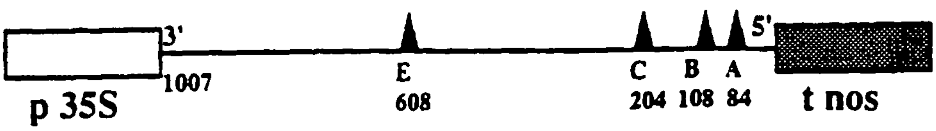

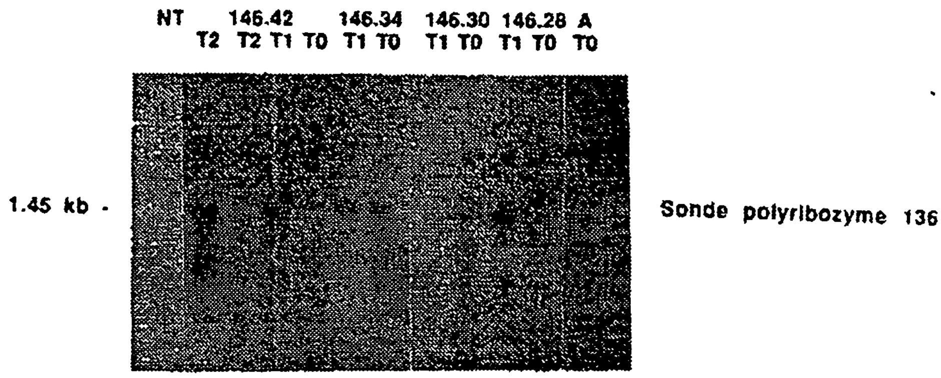


Légende
T0 : transformant primaire
T1 : descendant T1
T2 : descendant T2
A : lignée 141.1
NT : témoin non transformé

Figure 5 : ANALYSE NORTHERN DES PLANTES DE MELON TRANSGENIQUES (TRANSFORMANTS PRIMAIRES, DESCENDANTS T1 ET T2 DANS CERTAINS CAS) EXPRIMANT LE POLYRIBOZYME 136

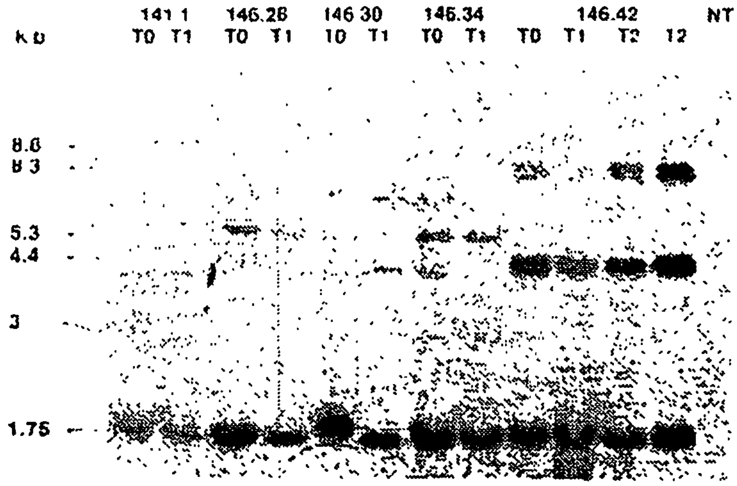
141 1
146.28
146 30
146.34
146.42
NT
Kb
T0 T1 T0 T1 T0 T1 T0 T1 T0 T1 T2 T2
8.6
8 3
5.3
4.4
3
1.75

Fig 6 a

# DIGESTION EcoRI

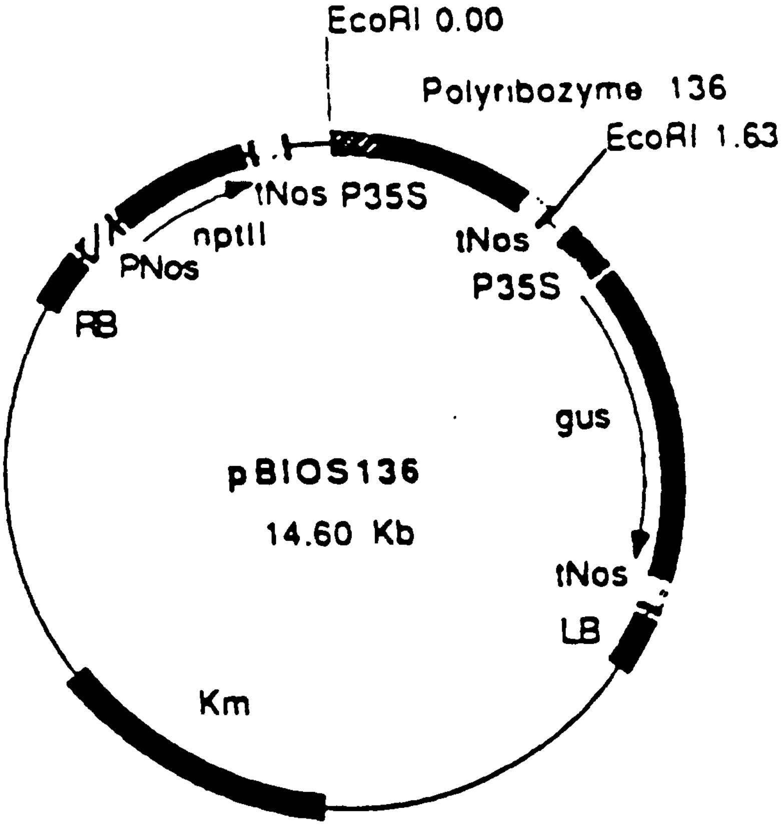


## Sonde polyribozyme 136

FIGURE 6B

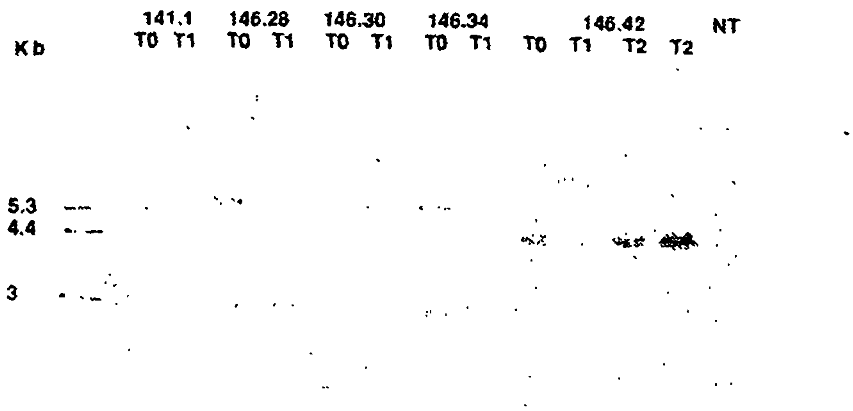
Kb
141.1
T0 T1
146.28
T0 T1
146.30
T0 T1
146.34
T0 T1
146.42
T0 T1 T2 T2
NT
5.3
4.4
3

Fig 6 c

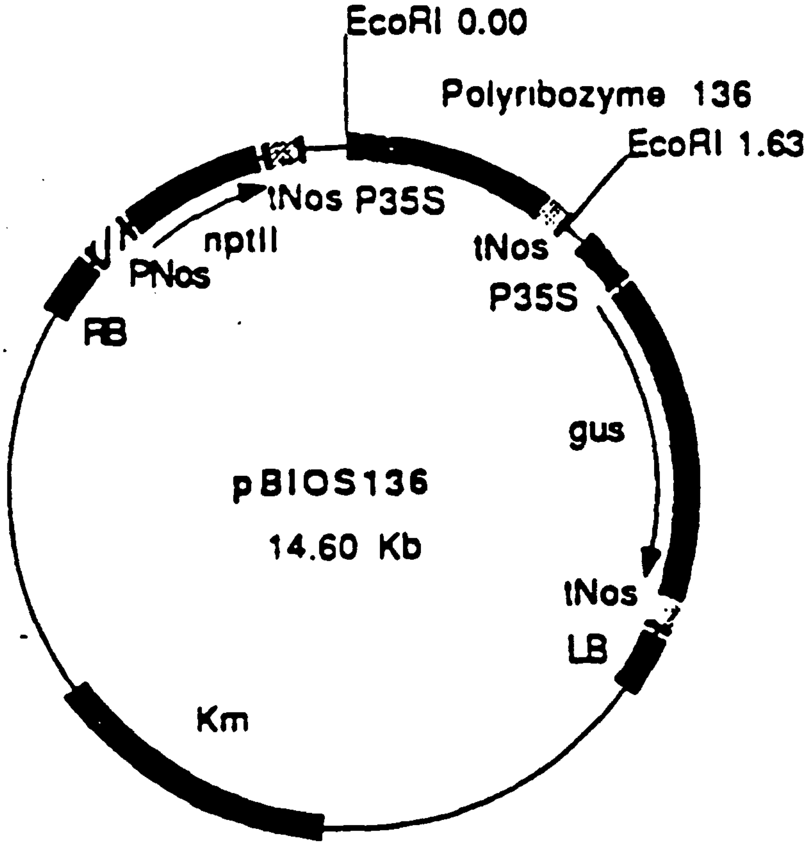


**Sonde npt.II**

FIGURE 6D

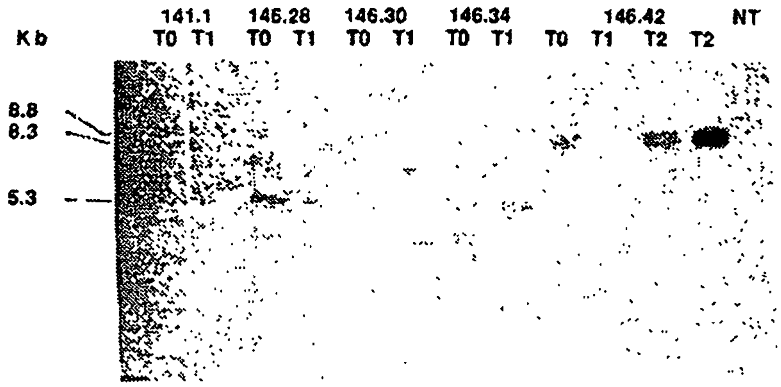
Kb
141.1
146.28
146.30
146.34
146.42
NT
T0 T1 T0 T1 T0 T1 T0 T1 T0 T1 T2 T2
8.8
8.3
5.3

Fig 6 E

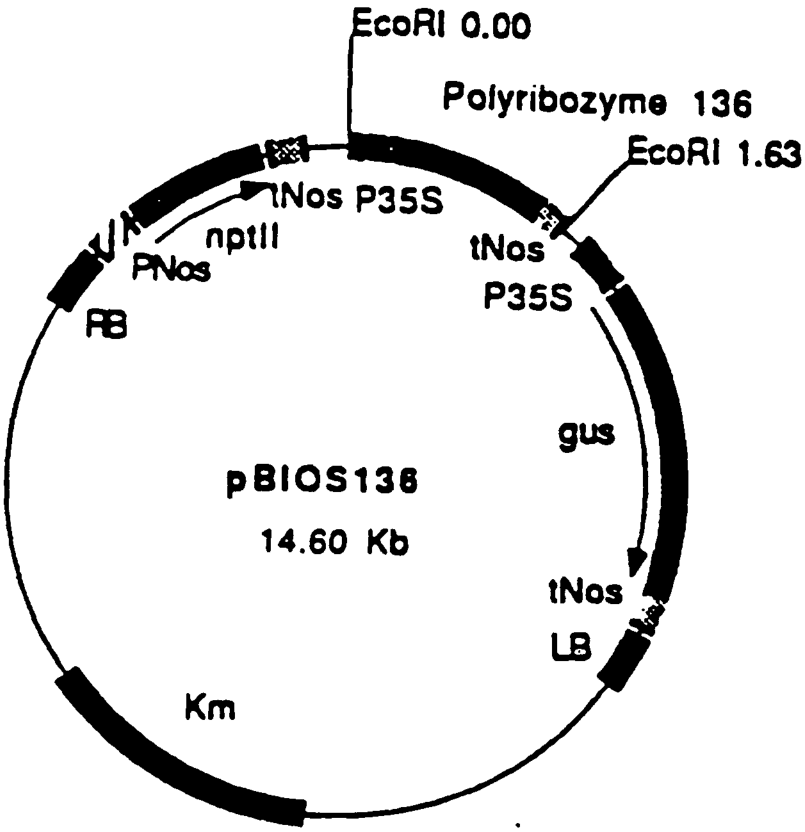


Sonde gus

FIGURE 6F

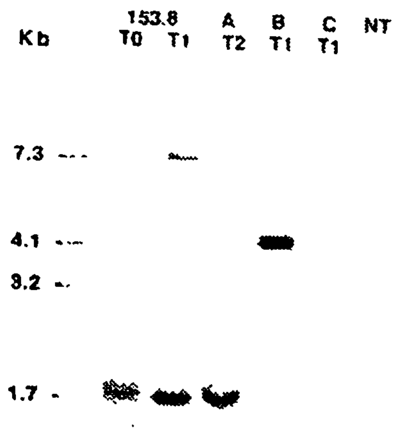


Fig 7A.

# DIGESTION EcoRI

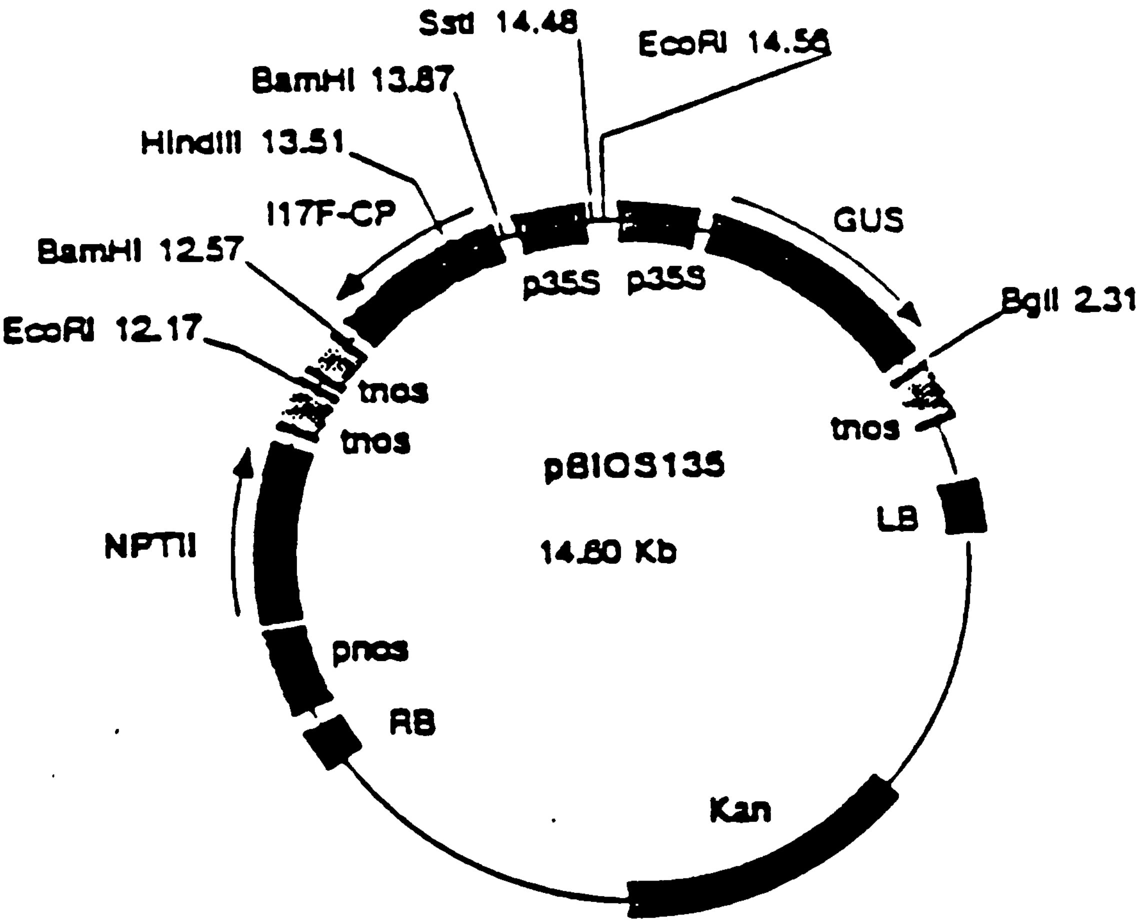


## Sonde I17F - cp

FIGURE 7B

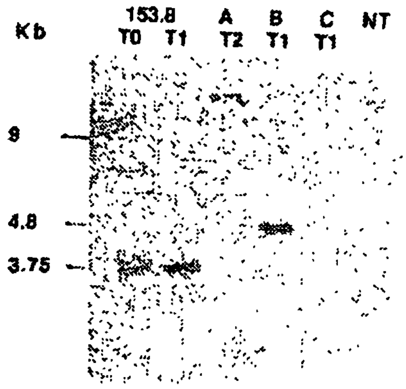


7C.

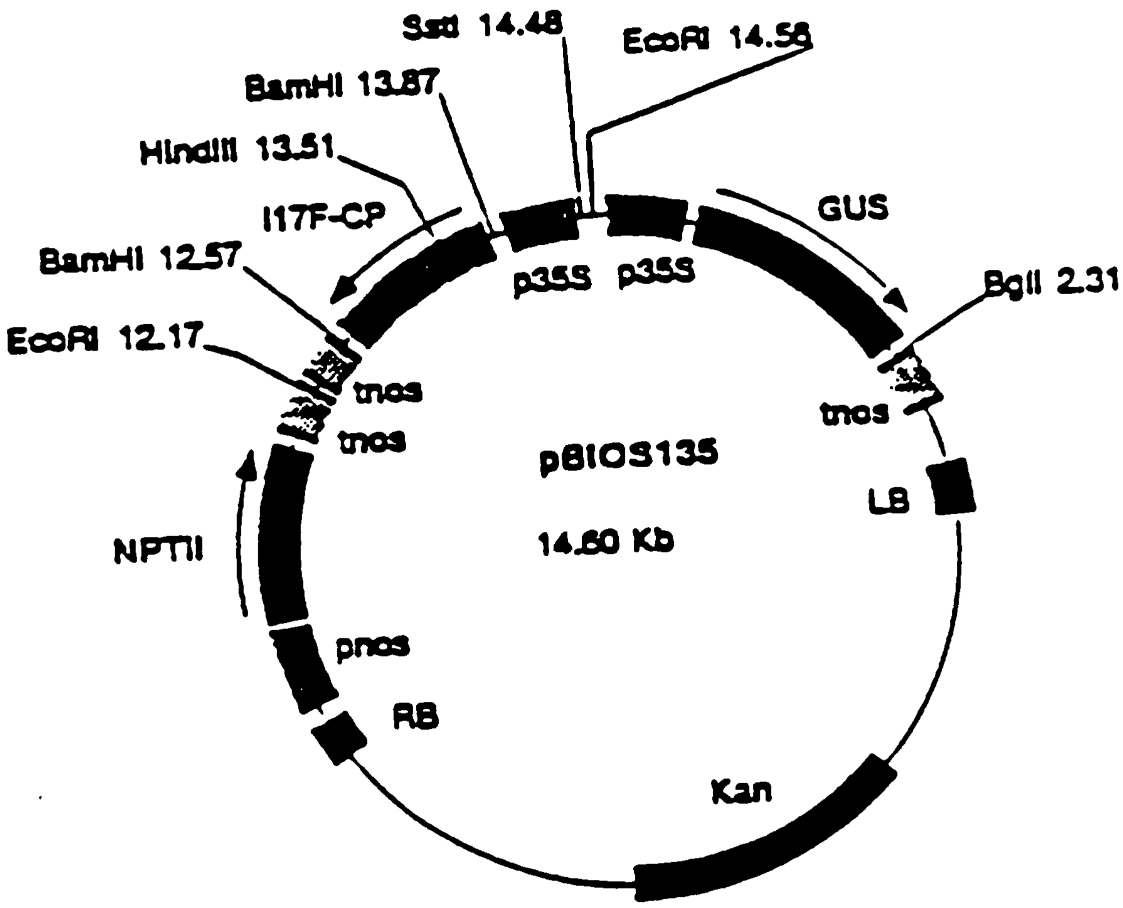


Sonde npt II

FIGURE 7D

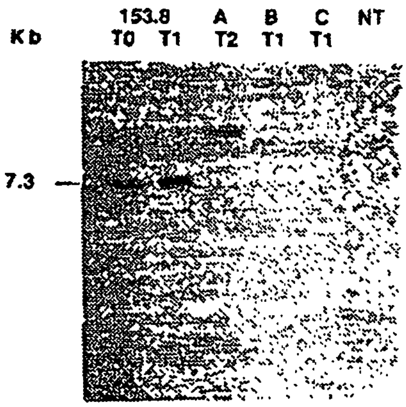


7E

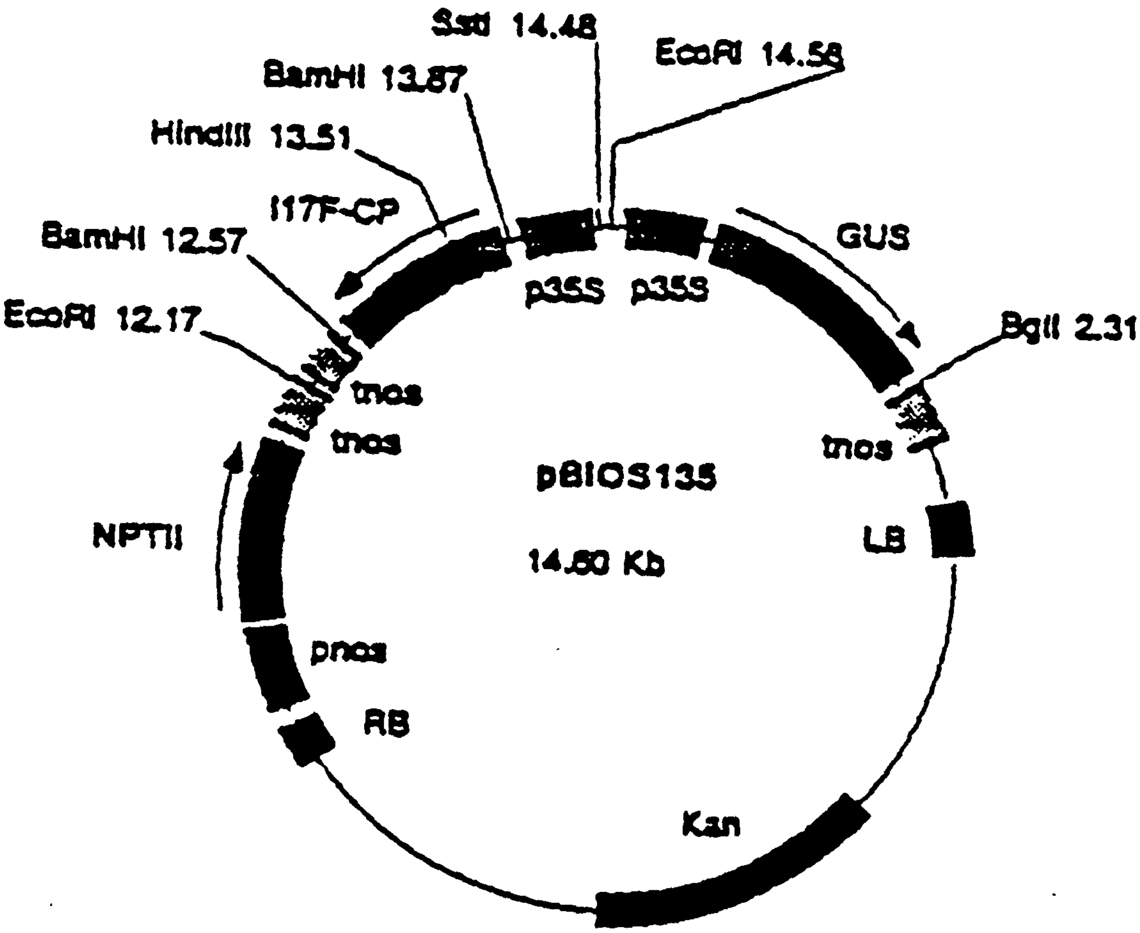


Sonde gus

FIGURE 7F

NT R A B C D E

27 kD -

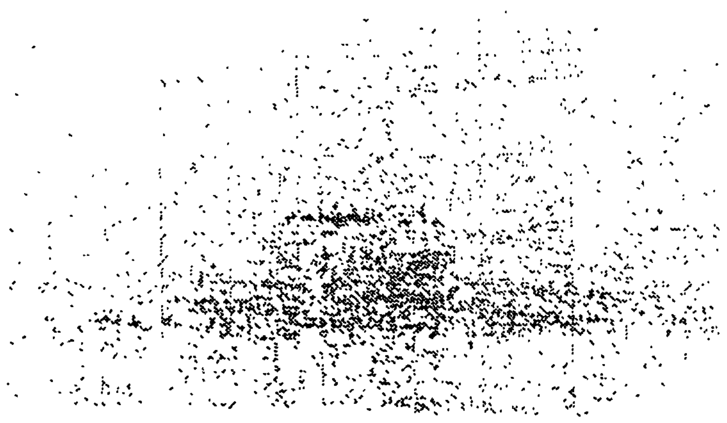

Légende :
A, B, C, D et E : transformants primaires de 5 lignées
NT : témoin non transformé
R : reconstruction avec 20 ng de CMV

Figure 6 : ANALYSE WESTERN DES PLANTES DE MELON TRANSGENIQUES (TRANSFORMANTS PRIMAIRES) TRANSFORMEES AVEC pBIOS135

FIGURE 9A

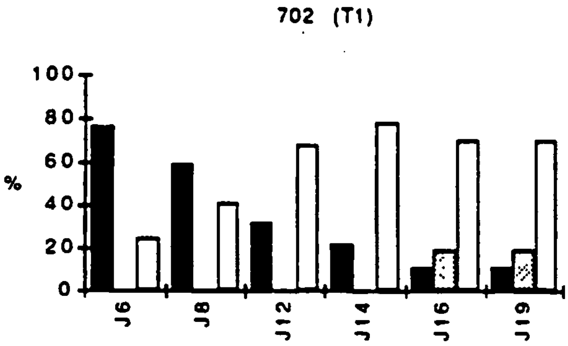


FIGURE 9B

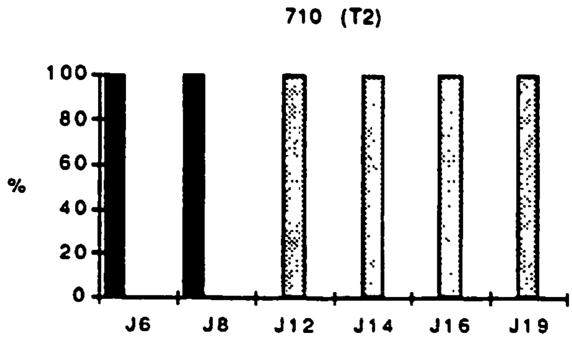


FIGURE 9C

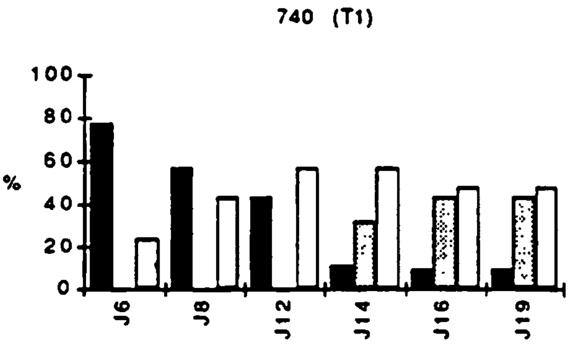


FIGURE 9D

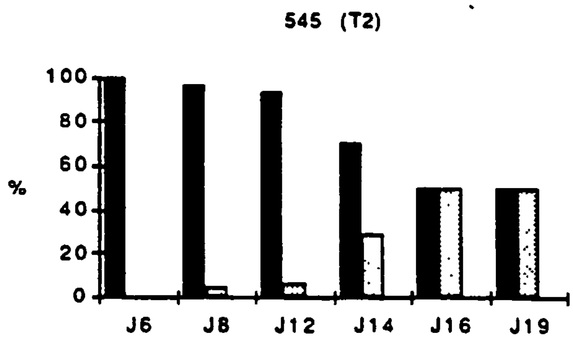


FIGURE 9E

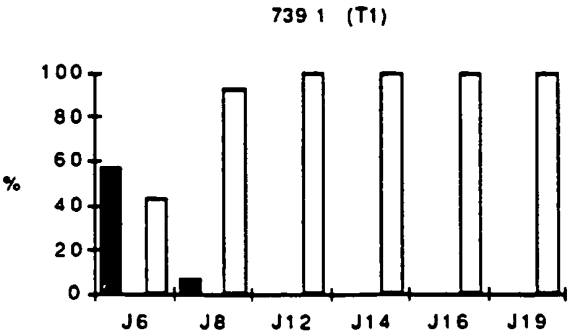

FIGURE 10A

730 1 (T1)

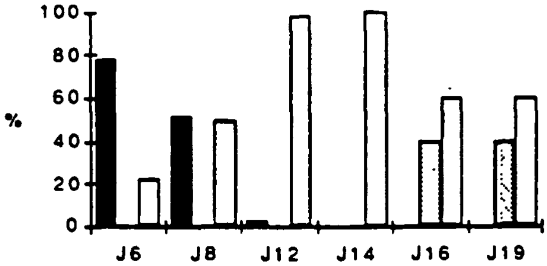


FIGURE 10B

734 1 (T1)

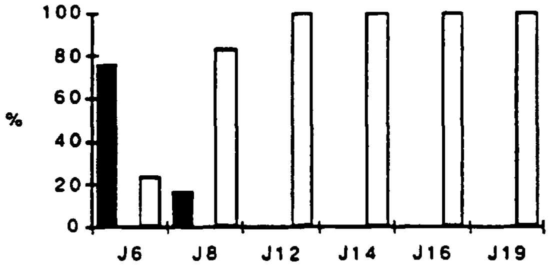


FIGURE 10C

539.2 (T2)

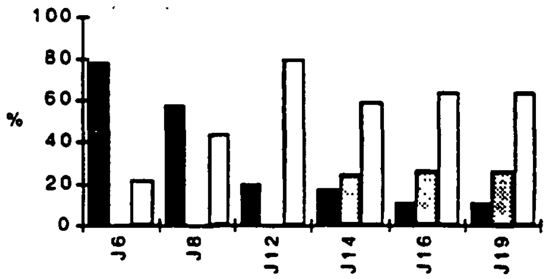


FIGURE 10D

735.1 (T1)

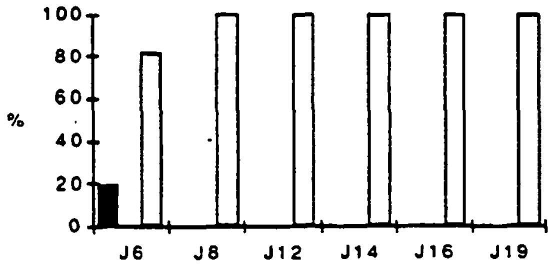


FIGURE 10E

755 1 (T1)

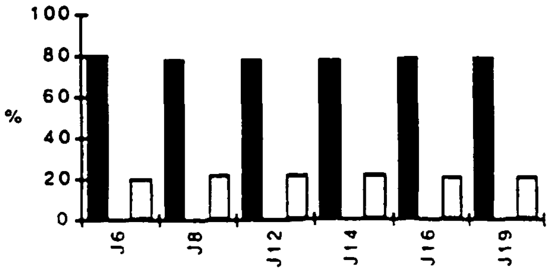


FIGURE 10F

540 (T2)